# EUROPEAN PATENT APPLICATION

(11) **EP 4 800 695 A2**
(43) Date of publication of application: **02.09.2026**
(21) Application number: 26192400.5
(22) Date of filing: 12.10.2023
(51) Int. Cl.: G16H 10/60

(54) **SECURE BROADCAST MESSAGING IN SUPPORT OF GLUCOSE MONITORING**

(30) Priority: 28.11.2022 US 202263385197 P
(62) Document number(s) of the earlier application(s) in accordance with Art. 76 EPC: 23804869.8 / 4 627 762
(71) Applicant: DexCom, Inc., San Diego, CA 92121 (US)
(72) Inventor: BARRERAS, Jorge R., San Diego, CA 92121 (US); PAUL, Nathanael Richard, San Diego, CA 92121 (US)
(74) Representative: Hoffmann Eitle

(57) **Abstract**

Techniques for performing application-layer security and communication over primary invitation channels are disclosed. In certain embodiments, analyte data is obtained from an analyte sensor electrically coupled to a sensor electronics module of an analyte sensor system. A secret key is established with a display device over one or more primary invitation channels. The analyte data is encrypted using the secret key. The encrypted analyte data is broadcast over the one or more primary invitation channels.

## Description

### CROSS-REFERENCE TO RELATED APPLICATIONS

This application claims the benefit of U.S. Application Serial No. 63/385,197 filed November 28, 2022, and hereby incorporated by reference in its entirety for all purposes.

### BACKGROUND

Diabetes is a metabolic condition relating to the production or use of insulin by the body. Insulin is a hormone that allows the body to use glucose for energy, or store glucose as fat.

Diabetes mellitus is a disorder in which the pancreas cannot create sufficient insulin (Type I or insulin dependent) and/or in which insulin is not effective (Type 2 or non-insulin dependent). In the diabetic state, the victim suffers from high blood sugar, which causes an array of physiological derangements (kidney failure, skin ulcers, or bleeding into the vitreous of the eye) associated with the deterioration of small blood vessels. A hypoglycemic reaction (low blood sugar) may be induced by an inadvertent overdose of insulin, or after a normal dose of insulin or glucose-lowering agent accompanied by extraordinary exercise or insufficient food intake.

Conventionally, a diabetic patient carries a self-monitoring blood glucose (SMBG) monitor, which may require uncomfortable finger pricking methods. Due to the lack of comfort and convenience, a diabetic will normally only measure his or her glucose level two to four times per day. Unfortunately, these time intervals are spread so far apart that the diabetic will likely be alerted to a hyperglycemic or hypoglycemic condition too late, sometimes incurring dangerous side effects as a result. In fact, it is unlikely that a diabetic will take a timely SMBG value, and further the diabetic will not know if his blood glucose value is going up (higher) or down (lower), due to limitations of conventional methods.

Consequently, a variety of non-invasive, transdermal (e.g., transcutaneous) and/or implantable sensors are being developed for continuously detecting and/or quantifying blood glucose values. Generally, in a diabetes management system, a transmitter associated with the sensor wirelessly transmits raw or minimally processed data for subsequent display and/or analysis at one or more display devices, which can include a mobile device, a server, or any other type of communication devices. A display device, such as a mobile device, may then utilize a trusted software application (e.g., approved and/or provided by the manufacturer of the sensor), which takes the raw or minimally processed data and provides the user with information about the user's blood glucose levels. Because diabetes management systems using such implantable sensors can provide more up-to-date information to users, they may reduce the risk of a user failing to regulate the user's blood glucose levels.

This background is provided to introduce a brief context for the summary and detailed description that follow. This background is not intended to be an aid in determining the scope of the claimed subject matter nor be viewed as limiting the claimed subject matter to implementations that solve any or all of the disadvantages or problems presented above.

### SUMMARY

Certain embodiments provide a computer-implemented method for communicating analyte data performed by a sensor electronics module of an analyte sensor system. The computer-implemented method includes obtaining analyte data from an analyte sensor electrically coupled to the sensor electronics module. The computer-implemented method also includes establishing a secret key with a display device over one or more primary invitation channels. The computer-implemented method further includes encrypting the analyte data using the secret key, and broadcasting the encrypted analyte data over the one or more primary invitation channels.

Certain embodiments provide a computer-implemented method for communicating analyte data performed by a display device. The computer-implemented method includes establishing a secret key with a sensor electronics module of an analyte sensor system over one or more primary invitation channels. The computer-implemented method also includes receiving encrypted analyte data from the sensor electronics module over the one or more primary invitation channels. The computer-implemented method further includes decrypting the encrypted analyte data using the secret key.

Certain embodiments provide a computer-implemented method for communicating analyte data performed by a display device. The computer-implemented method includes establishing a secret key with a sensor electronics module of an analyte sensor system over one or more primary invitation channels. The computer-implemented method also includes receiving, at a first point in time, encrypted first analyte data from the sensor electronics module over the one or more primary invitation channels. The computer-implemented method also includes decrypting the encrypted first analyte data using the secret key. The computer-implemented method also includes receiving, at a second point in time subsequent to the first point in time, encrypted second analyte data associated with the analyte sensor system from a server. The computer-implemented method further includes decrypting the encrypted second analyte data using the secret key.

Certain embodiments provide a computer-implemented method for communicating analyte data performed by an intermediary device. The computer-implemented method includes receiving an indication of a secret key from a server, the secret key being associated with a sensor electronics module of an analyte sensor system. The computer-implemented method also includes receiving encrypted analyte data from the sensor electronics module over one or more primary invitation channels. The computer-implemented method also includes decrypting the encrypted analyte data using the secret key. The computer-implemented method also includes displaying the decrypted analyte data. The computer-implemented method also includes re-encrypting the decrypted analyte data using the secret key, and transmitting the re-encrypted analyte data to the server over a secure channel established between the intermediary device and the server.

Certain embodiments provide a computer-implemented method for communicating analyte data performed by an intermediary device. The computer-implemented method includes receiving encrypted analyte data from a sensor electronics module of an analyte sensor system over one or more primary invitation channels. The computer-implemented method also includes transmitting the encrypted analyte data to a server over a secure channel established between the intermediary device and the server.

Certain embodiments provide a computer-implemented method for communicating analyte data performed by a server. The computer-implemented method includes establishing a secret key with a first intermediary device. The computer-implemented method also includes transmitting an indication of the secret key to at least one second intermediary device. The computer-implemented method also includes receiving encrypted analyte data associated with a sensor electronics module of an analyte sensor system from at least one of the first intermediary device or the second intermediary device, the analyte data being encrypted with the secret key. The computer-implemented method further includes transmitting the encrypted analyte data to a display device.

Certain embodiments provide an analyte sensor system. The analyte sensor system includes an analyte sensor and a sensor electronics module electrically coupled to the analyte sensor. The sensor electronics module is configured to perform an operation. The operation includes obtaining analyte data from the analyte sensor. The operation also includes establishing a secret key with a display device over one or more primary invitation channels. The operation also includes encrypting the analyte data using the secret key. The operation further includes broadcasting the encrypted analyte data over the one or more primary invitation channels.

Certain embodiments provide a computer-implemented method for communicating analyte data performed by a sensor electronics module of an analyte sensor system. The computer-implemented method includes obtaining analyte data from an analyte sensor electrically coupled to the sensor electronics module. The computer-implemented method also includes establishing a secret key with a display device, based on executing a cryptographic key exchange algorithm at an application layer of a communication protocol stack at the sensor electronics module. The computer-implemented method also includes encrypting the analyte data using the secret key. The computer-implemented method further includes transmitting the encrypted analyte data.

Certain embodiments provide a system. The system includes an analyte sensor, a first display device, a server, a sensor electronics module, and a second display device. The sensor electronics module is configured to obtain analyte data from the analyte sensor, establish a secret key with the first display device over one or more primary invitation channels, encrypt the analyte data using the secret key, and broadcast the encrypted analyte data over the one or more primary invitation channels. The second display device is configured to receive the encrypted analyte data broadcast over the one or more primary invitation channels and transmit the encrypted analyte data to the server over a secure channel established between the second display device and the server. The server is configured to transmit the encrypted analyte data to the first display device over a secure channel established between the first display device and the server. The first display device is configured to receive the encrypted analyte data transmitted from the server, decrypt the encrypted analyte data using the secret key, and display the decrypted analyte data.

### BRIEF DESCRIPTION OF THE DRAWINGS

**FIG. 1A** illustrates an example diabetes management system, according to certain embodiments disclosed herein.
**FIG. 1B** illustrates the example diabetes management system of **FIG. 1A** in more detail, according to certain embodiments disclosed herein.
**FIG. 2** illustrates an example communication protocol architecture, according to certain embodiments disclosed herein.
**FIG. 3** is a sequence diagram illustrating example operations performed by an analyte sensor system and a display device, according to certain embodiments disclosed herein.
**FIG. 4** is a sequence diagram illustrating example operations performed by an analyte sensor system, one or more display devices, and a server system, according to certain embodiments disclosed herein.
**FIG. 5** is a sequence diagram illustrating example operations performed by an analyte sensor system, an intermediary device, a display device, and a server system, according to certain embodiments disclosed herein.
**FIG. 6** is a sequence diagram illustrating example operations performed by an analyte sensor system, one or more intermediary devices, a display device, and a server system, according to certain embodiments disclosed herein.
**FIG. 7** is a sequence diagram illustrating example operations performed by an analyte sensor system, an intermediary device, a display device, and a server system, according to certain embodiments disclosed herein.
**FIG. 8** is a sequence diagram illustrating example operations performed by an analyte sensor system and an intermediary device, according to certain embodiments disclosed herein.
**FIG. 9** is a sequence diagram illustrating example operations performed by an analyte sensor system, one or more devices, and a server system, according to certain embodiments disclosed herein.
**FIG. 10** is a sequence diagram illustrating example operations performed by an analyte sensor system, a display device, and a server system, according to certain embodiments disclosed herein.
**FIG. 11** is a flow diagram illustrating example operations for communicating analyte information by an analyte sensor system, according to certain embodiments disclosed herein.
**FIG. 12** is a flow diagram illustrating example operations for communicating analyte information by a display device, according to certain embodiments disclosed herein.
**FIG. 13** is a flow diagram illustrating example operations for communicating analyte information by an intermediary device, according to certain embodiments disclosed herein.

### DETAILED DESCRIPTION OF CERTAIN INVENTIVE EMBODIMENTS

In a continuous glucose monitor (CGM), a glucose sensor typically measures glucose levels of a patient and communicates the raw sensor measurements to a transmitter, which then transmits corresponding glucose values to a patient's display device, such as a mobile phone. In order to connect with the display device for the initial time, the transmitter is configured to transmit one or more invitation or broadcast or advertisement (also referred to as inviting or broadcasting or advertising, respectively) packets to the display device through one or more primary advertisement (hereinafter "invitation") channels.

In response to the invitation packets, the display device and the transmitter may engage in a connection request/response exchange through the one or more primary invitation channels to establish a connection. Subsequently, the display device and the transmitter may engage in authentication, pairing, and/or bonding through one or more data channels. In certain cases, the one or more primary invitation channels represent three out of forty different frequency channels, and the one or more data channels represent the remaining thirty-seven of the forty channels. The three frequency channels are designated for transmission of primary invitation packets, and the thirty-seven frequency channels are designated for transmission of data packets.

After bonding, the two devices exchange data (e.g., glucose values), and then disconnect. Once the transmitter and the display device have paired and bonded, at each of the devices, information about the other device and the bond that has been created with the other device is stored. For example, at the transmitter, the display device is added to a "targeted device list," where information about the bond that has been created with the display device is stored and then used for reconnections. As a result, pairing and bonding will not be necessary during reconnections.

Reconnections may occur periodically, such as every five minutes so that the transmitter can provide updated glucose values to the display devices. During a reconnection, the link between the transmitter and the display device for exchanging data is secured using the information stored about the bond (e.g., authentication, encryption, and other similar information).

Typically, the transmitter's communication stack (e.g., communication protocol stack, such as Bluetooth Low Energy (BLE) stack) includes an application layer and a lower protocol layer (e.g., BLE layer). Data, such as glucose value(s), is generated at the application layer of the transmitter's communication stack, while data connections/disconnections, security, pairing, bonding, etc., are all handled at the lower protocol layer. However, implementing data security (e.g., integrity, authentication, and encryption) at the lower layer of the communication stack may introduce a number of technical issues, including, for example, resource inefficiencies and signal loss issues.

First, as described above, each time the transmitter and the display device wish to exchange data, they are required by their lower protocol layer to connect, exchange data, and disconnect. This connection/disconnection routine occurs regardless of whether it is the initial time the transmitter and the display device are connecting or whether it is during a reconnection. However, performing this connection/disconnection repeatedly is resource inefficient, as each connection/disconnection routine consumes time as well as compute, battery, and communication resources, all of which can cause increased battery consumption of the transmitter.

Second, as described above, the transmitter may be required to add a display device to a "targeted device list" after a first successful connection. While such a "targeted device list" is conventionally used to reduce the need to perform pairing and bonding during a reconnection with a display device on the "targeted device list," the transmitter still has to spend compute and storage resources to create and maintain a "targeted device list" and store information about the display device for use during reconnections.

Third, as discussed, when the devices are connecting for the initial time, the lower protocol layer requires that pairing and bonding take place between the devices, which may be resource inefficient. In particular, engaging in pairing and bonding consumes time as well as compute, storage, battery, and communication resources, all of which can cause increased battery consumption of the transmitter.

Fourth, currently, when a message is generated at the application layer of the transmitter, prior to transmission to a display device over the air, the message is automatically encrypted at the lower protocol layer. However, if the message has to be re-sent (e.g., due to a transmission error), prior to re-transmission, the lower protocol layer will have to re-encrypt the same message because the previously re-encrypted message is not cached. This re-encryption of the same message is computationally expensive and consumes power, which can also lead to increased battery consumption of the transmitter.

There are many additional requirements that are required by the lower protocol layer to be met in relation to the communication between the transmitter and display device that are similarly resource inefficient. Such resource inefficiencies can lead to increased battery consumption of the transmitter.

To address the aforementioned challenges associated with providing data security at the protocol layer of a communication stack, certain embodiments described herein provide improved techniques for communicating analyte data between a transmitter of an analyte sensor system and a display device. More specifically, in certain embodiments, the transmitter and the display device are configured to perform encryption, data integrity, and/or authentication at the application layer of a communication protocol stack, as opposed to at a lower protocol layer.

However, note that even if data security is provided at the application layer instead of the lower protocol layer, in certain situations, if data is exchanged through any of the data channels described above, then the lower protocol layer is configured to automatically perform pairing, bonding, connecting, and disconnecting as well as provide an additional layer of protocol-based data security. Therefore, in order to circumvent the need to pair, bond, connect, and disconnect through the lower protocol layer, in certain embodiments, the display device and the transmitter are configured to only communicate over the three primary invitation channels. As such, by performing application-layer security and communicating over the primary invitation channels, the transmitter and display device can securely exchange analyte information without the need to pair, bond, connect, disconnect, as well as the need to use any security protocols offered by the lower protocol layer.

Advantageously, performing application-layer security and communicating over the three primary invitation channels using the techniques described herein can reduce consumption of resources (e.g., computer resources, memory resources, etc.) by the transmitter, since the transmitter can avoid repeatedly connecting and disconnecting to send analyte information. Reducing consumption of resources, in turn, reduces battery consumption, which is critical to extending the operational life of the transmitter.

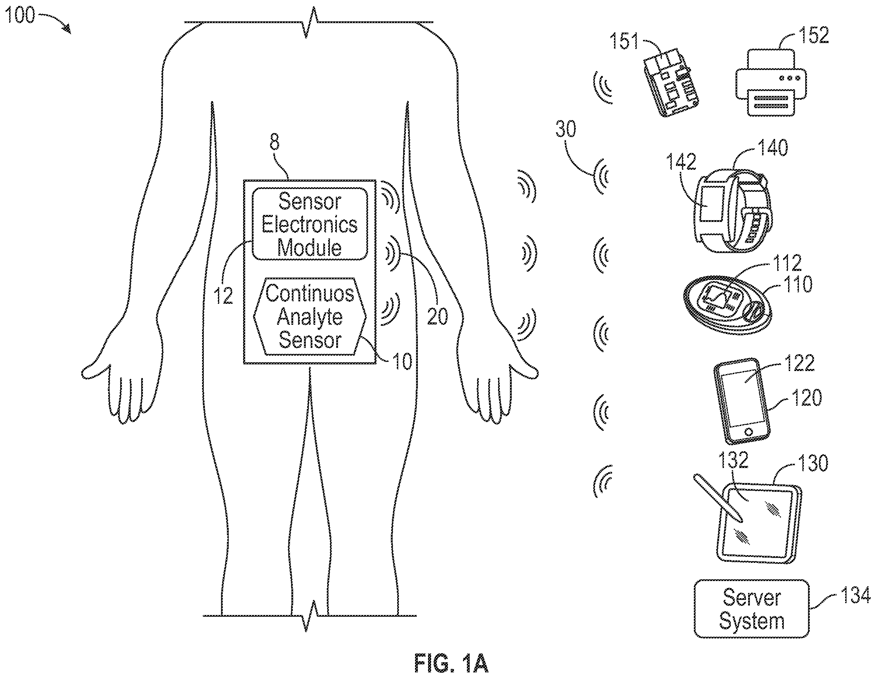

The techniques described herein for performing application-layer security and communication over primary invitation channels are described more fully herein with respect to **FIGs. 1A-1B** and **2-13** below. Note that, hereinafter, although certain embodiments described herein refer to an analyte sensor system performing one or more techniques described herein for application-layer security and communication over primary invitation channels, it is the transmitter (also referred to as the sensor electronics module) in the analyte sensor system that performs the techniques described herein for application-layer security and communication over primary invitation channels. Additionally, note that although certain embodiments herein are described with respect to the management of diabetes, a glucose sensor system, and the transmission of glucose measurement between the devices, the protocols and techniques described herein are similarly applicable to any type of health management system that includes any type of analyte sensor (e.g., lactate sensor, ketone sensor, etc.).

### EXAMPLE ANALYTE SENSOR SYSTEM

**FIG. 1A** depicts a disease management system 100 ("system 100"), such as a diabetes management system, that may be used in connection with certain embodiments of the present disclosure. Certain such embodiments may involve performing application-layer security and communication over primary invitation channels to reduce resource consumption by the transmitter of an analyte sensor system. System 100 depicts aspects of analyte sensor system 8 (hereinafter "SS 8") that may be communicatively coupled to display devices 110, 120, 130, and 140, to intermediary devices 151 and 152, and/or to server system 134.

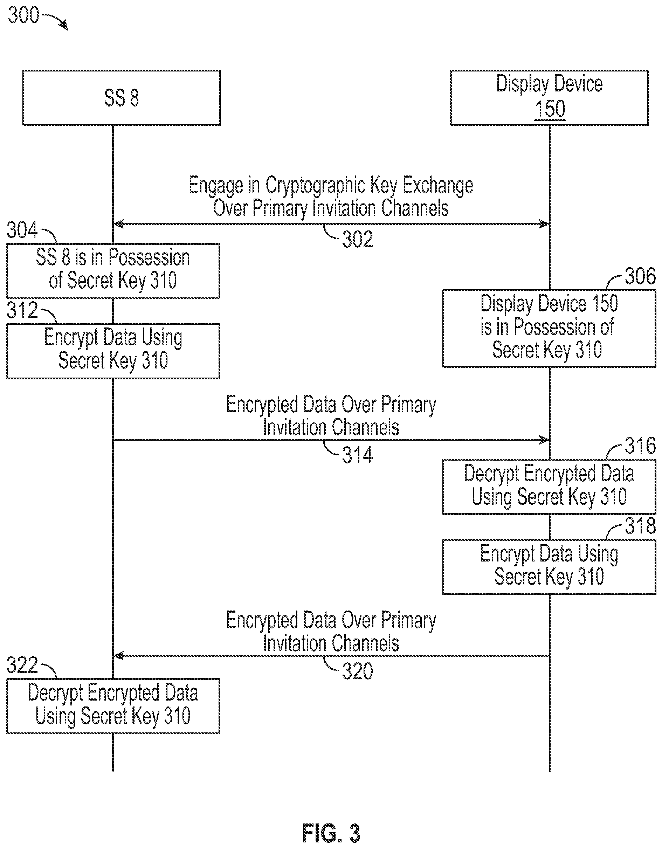

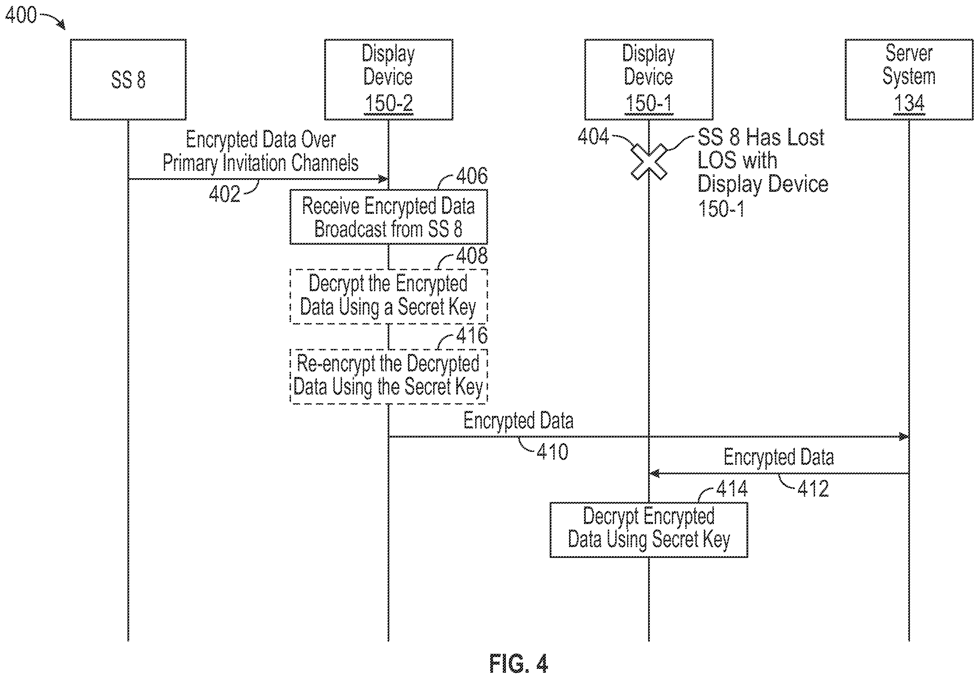

In certain embodiments, SS 8 is provided for measurement of an analyte in a host or a user. By way of an overview and an example, SS 8 may be implemented as an encapsulated microcontroller that makes sensor measurements, generates analyte data (e.g., by calculating values for continuous glucose monitoring data), and engages in wireless communications (e.g., via Bluetooth and/or other wireless protocols) to send such data to remote devices, such as display devices 110, 120, 130, 140, intermediary devices 151 and 152, and/or server system 134. Paragraphs [0137]-[0140] and FIGs. 3A, 3B, and 4 of U.S. App. No. 2019/0336053 further describe an on-skin sensor assembly that, in certain embodiments, may be used in connection with SS 8. Paragraphs [0137]-[0140] and FIGs. 3A, 3B, and 4 of U.S. App. No. 2019/0336053 are incorporated herein by reference.

In certain embodiments, SS 8 includes an analyte sensor electronics module 12 and an analyte sensor 10 associated with analyte sensor electronics module 12. In certain embodiments, analyte sensor electronics module 12 includes electronic circuitry associated with measuring and processing analyte sensor data or information, including algorithms associated with processing and/or calibration of the analyte sensor data/information. Analyte sensor electronics module 12 may be physically/mechanically connected to analyte sensor 10 and can be integral with (i.e., non-releasably attached to) or releasably attachable to analyte sensor 10.

Analyte sensor electronics module 12 may also be electrically coupled to analyte sensor 10, such that the components may be electromechanically coupled to one another (e.g., (a) prior to insertion into a patient's body, or (b) during the insertion into the patient's body). Analyte sensor electronics module 12 may include hardware, firmware, and/or software that enable measurement and/or estimation of levels of the analyte in a host/user via analyte sensor 10 (e.g., which may be/include a glucose sensor). For example, analyte sensor electronics module 12 can include one or more potentiostats, a power source for providing power to analyte sensor 10, other components useful for signal processing and data storage, and a telemetry module for transmitting data from the sensor electronics module to one or more display devices. Electronics can be affixed to a printed circuit board (PCB) within SS 8, or platform or the like, and can take a variety of forms. For example, the electronics can take the form of an integrated circuit (IC), such as an Application-Specific Integrated Circuit (ASIC), a microcontroller, a processor, and/or a state machine.

Analyte sensor electronics module 12 may include sensor electronics that are configured to process sensor information, such as sensor data, and generate transformed sensor data and displayable sensor information. Examples of systems and methods for processing sensor analyte data are described in more detail herein and in U.S. Pat. Nos. 7,310,544 and 6,931,327 and U.S. Patent Publication Nos. 2005/0043598, 2007/0032706, 2007/0016381, 2008/0033254, 2005/0203360, 2005/0154271, 2005/0192557, 2006/0222566, 2007/0203966 and 2007/0208245, all of which are incorporated herein by reference in their entireties.

Analyte sensor 10 is configured to measure a concentration or level of the analyte in the host. The term analyte is further defined by paragraph [0117] of U.S. App. No. 2019/0336053. Paragraph [0117] of U.S. App. No. 2019/0336053 is incorporated herein by reference. In some embodiments, analyte sensor 10 comprises a continuous glucose sensor, such as a subcutaneous, transdermal (e.g., transcutaneous), or intravascular device. In some embodiments, analyte sensor 10 can analyze a plurality of intermittent blood samples. Analyte sensor 10 can use any method of glucose-measurement, including enzymatic, chemical, physical, electrochemical, spectrophotometric, polarimetric, calorimetric, iontophoretic, radiometric, immunochemical, and the like. Additional details relating to a continuous glucose sensor are provided in paragraphs [0072]-[0076] of U.S. App. No. 13/827,577. Paragraphs [0072]-[0076] of U.S. App. No. 13/827,577 are incorporated herein by reference. In certain embodiments, analyte sensor 10 may be configured to sense multiple analytes (e.g., glucose, potassium, lactate, and/or others).

With further reference to **FIG. 1A****,** display devices 110, 120, 130, and/or 140 can be configured for displaying (and/or alarming) displayable sensor information that may be transmitted by sensor electronics module 12 (e.g., in a customized data package that is transmitted to the display devices based on their respective preferences). Each of display devices 110, 120, 130, or 140 may respectively include a display such as touchscreen display 112, 122, 132, and/or 142 for displaying sensor information and/or analyte data to a user and/or receiving inputs from the user. For example, a graphical user interface (GUI) may be presented to the user for such purposes. In certain embodiments, the display devices may include other types of user interfaces such as voice user interface instead of or in addition to a touchscreen display for communicating sensor information to the user of the display device and/or receiving user inputs. In certain embodiments, one, some, or all of display devices 110, 120, 130, 140 may be configured to display or otherwise communicate the sensor information as it is communicated from sensor electronics module 12 (e.g., in a data package that is transmitted to respective display devices), without any additional prospective processing required for calibration and/or real-time display of the sensor data.

The plurality of display devices 110, 120, 130, 140 depicted in **FIG. 1A** may include a custom or proprietary display device, for example, analyte display device, especially designed for displaying certain types of displayable sensor information associated with analyte data received from sensor electronics module 12 (e.g., a numerical value and/or an arrow, in certain embodiments). In certain embodiments, one of the plurality of display devices 110, 120, 130, 140 includes a smartphone, such as display device 120, based on an Android, iPhone Operating System (iOS), or another operating system configured to display a graphical representation of the continuous sensor data (e.g., including current and/or historic data). In certain embodiments, disease management system 100 further includes a medical delivery device (e.g., an insulin pump or pen). Sensor electronics module 12 may be configured to transmit sensor information and/or analyte data to the medical delivery device. The medical delivery device (not shown) may be configured to administer a certain dosage of insulin or another medicament to the user based on the sensor information and/or analyte data (e.g., which may include a recommended insulin dosage) received from the sensor electronics module 12.

With further reference to **FIG. 1A****,** intermediary devices 151 and/or 152 can be configured for receiving and/or forwarding sensor information that may be transmitted by sensor electronics module 12 and/or display devices 110, 120, 130, and/or 140. Although not shown, one or more of intermediary devices 151 and 152 may include a display such as touchscreen display for displaying sensor information and/or analyte data to a user and/or receiving inputs from the user. In certain embodiments, one, some, or all of intermediary devices 151 and 152 may be configured to display or otherwise communicate the sensor information as it is communicated from sensor electronics module 12 (e.g., in a data package that is transmitted to respective display devices), without any additional prospective processing required for calibration and/or real-time display of the sensor data.

The plurality of intermediary devices 151 and 152 depicted in **FIG. 1A** may include a custom or proprietary intermediary device, for example, a Raspberry Pi device, especially designed for displaying and/or communicating certain types of displayable sensor information associated with analyte data received from sensor electronics module 12 (e.g., a numerical value and/or an arrow, in certain embodiments). In certain embodiments, one of the plurality of intermediary devices 151 and 152 includes a wireless printer, such as intermediary device 152. In certain embodiments, one of the plurality of intermediary devices 151 and 152 includes a Raspberry Pi device, such as Raspberry Pi device 151. Compared to display devices 110, 120, 130, and/or 140, one or more of the intermediary devices 151 and 152 may have limited capabilities. For example, the intermediary devices 151 and 152 may be configured to just communicate (e.g., send/receive) information, such as sensor information and/or analyte data. Note, however, that in certain embodiments, at least one or more of the intermediary devices 151 and 152 may be configured to display sensor information and/or analyte data to a user and/or receive inputs from the user. Although a Raspberry Pi device and wireless printer are used as examples of intermediary devices, note that any device that is configured to wirelessly communicate using a wireless communication protocol (e.g., WiFi, Bluetooth, cellular communication, etc.) can be configured as an intermediary device.

Server system 134 may be used to directly or indirectly collect analyte data from SS 8, the plurality of intermediary devices, and/or the plurality of display devices, for example, to perform analytics thereon, generate universal or individualized models for analyte levels and profiles, provide services or feedback, including from individuals or systems remotely monitoring the analyte data, perform or assist SS 8, intermediary device 160, and display device 150 with secure communication of analyte information, etc., according to the embodiments described herein. Note that, in certain embodiments, server system 134 may be representative of multiple systems or computing devices that perform the functions of server system 134 (e.g., in a distributed manner). In certain embodiments, the server system 134 may be deployed in a cloud computing environment, which may be a local cloud environment or public cloud environment.

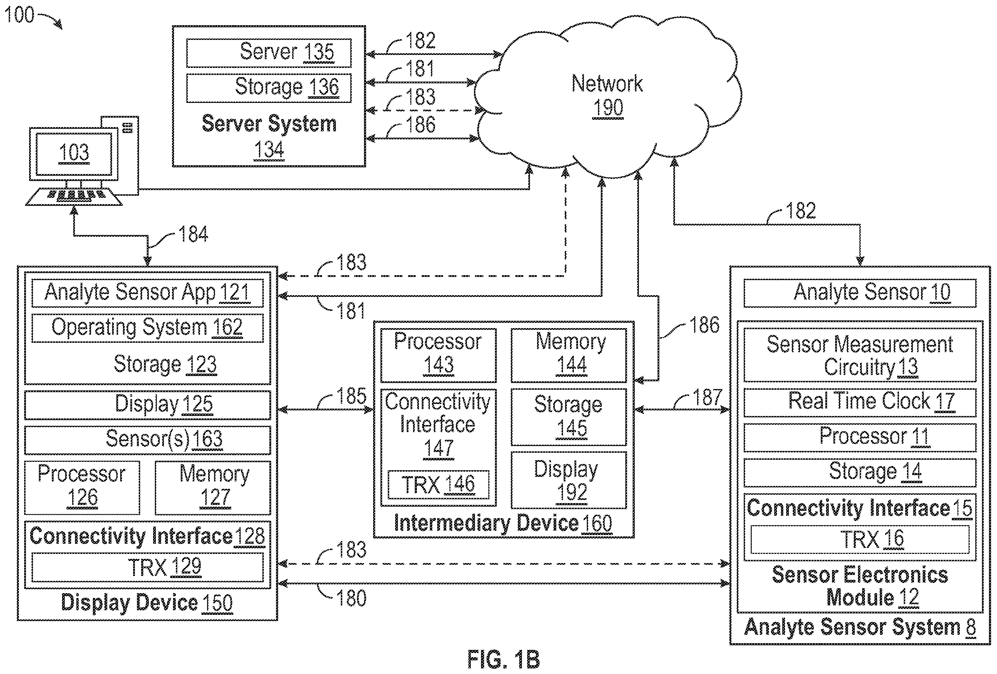

**FIG. 1B** illustrates a more detailed view of system 100 including a display device 150 that is communicatively coupled to SS 8. In certain embodiments, display device 150 may be any one of display devices 110, 120, 130, and 140 of **FIG. 1A****.** The communication path between SS 8 and display device 150 is shown as communication path 180. In certain embodiments, SS 8 and display device 150 are configured to wirelessly communicate over communication path 180 using low range and/or distance wireless communication protocols. Examples of low range and/or distance wireless communication protocols include Bluetooth and Bluetooth Low Energy (BLE) protocols. In certain embodiments, other short range wireless communications may include Near Field Communications (NFC), radio frequency identification (RFID) communications, IR (infra red) communications, and optical communications, as illustrative, non-limiting examples. In certain embodiments, wireless communication protocols other than low range and/or distance wireless communication protocols may be used for communication path 180, such as WiFi Direct. Display device 150 is also configured to connect to network 190 (e.g., local area network (LAN), wide area network (WAN), the Internet, etc.). For example, display device 150 may connect to network 190 via a wired (e.g., Ethernet) or wireless (e.g., wireless LAN (WLAN), wireless WAN, cellular, Mesh network, personal area network (PAN) etc.) interface. Display device 150 is able to communicate with server system 134 through network 190. The communication path between display device 150 and server system 134 is shown as communication path 181 via network 190.

Note that, in certain embodiments, SS 8 may be able to independently (c.g., wirelessly) communicate with server system 134 through network 190. An independent communication path between SS 8 and server system 134 is shown as communication path 182. However, in certain other embodiments, SS 8 may not be configured with the necessary hardware/software to establish, for example, an independent wireless communication path with server system 134 through network 190. In such embodiments, SS 8 may communicate with server system 134 through display device 150. An indirect or pass-through communication path between SS 8 and server system 134 is shown as communication path 183.

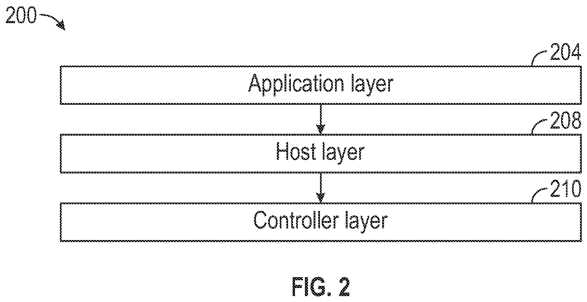

In embodiments where display device 150 is a proprietary display device, such as display device 110 designed specifically for the communication of analyte data, display device 150 may not be configured with the necessary hardware/software for independently connecting to network 190. Instead, in certain such embodiments, display device 150 is configured to establish a wired or wireless communication path 184 (e.g., through a Universal System Bus (USB) connection) with computer device 103, which is configured to communicate with server system 134 through network 190. For example, computer device 103 may connect to network 190 via a wired (e.g., Ethernet) or wireless (e.g., WLAN, wireless WAN, cellular, etc.) interface. Note that in the embodiments described in relation to **FIGs. 2-13****,** unless otherwise noted, display device 150 is assumed to be capable of independently communicating with server system 134 through network 190, independent of computer device 103.

In certain embodiments, system 100 additionally includes intermediary device 160, which may be any one of intermediary devices 151 and 152 of **FIG. 1A****.** The communication path between intermediary device 160 and display device 150 is shown as communication path 185, and the communication path between intermediary device 160 and SS 8 is shown as communication path 187. In certain embodiments, SS 8 and intermediary device 160 are configured to wirelessly communicate over communication path 187 using low range and/or distance wireless communication protocols and display device 150 and intermediary device are configured to wirelessly communicate over communication path 185 using low range and/or distance wireless communication protocols. Intermediary device 160 is also configured to connect to network 190. For example, intermediary device 160 may connect to network 190 via a wireless (e.g., WLAN, wireless WAN, cellular, Mesh network, personal area network (PAN) etc.) interface. Intermediary device 160 is able to communicate with server system 134 through network 190. The communication path between intermediary device 160 and server system 134 is shown as communication path 186 via network 190.

System 100 additionally includes server system 134, which in turn includes server 135 that is coupled to storage 136 (e.g., one or more computer storage systems, cloud-based storage systems and/or services, etc.). In certain embodiments, server system 134 may be located or execute in a public or private cloud. In certain embodiments, server system 134 is located or executes on-premises ("on-prem"). As discussed, server system 134 is configured to receive, collect, and/or monitor information, including analyte data and related information, as well as encryption/authentication information from SS 8 and/or display device 150. Such information may include input responsive to the analyte data or input (e.g., the user's glucose measurements and other physiological/behavioral information) received in connection with an analyte monitoring or sensor application running on SS 8 or display device 150. This information may be stored in storage 136 and may be processed, such as by an analytics engine capable of performing analytics on the information. An example of an analyte sensor application that may be executable on display device 150 is analyte sensor application 121, further described below.

In certain embodiments, server system 134 at least partially directs communications between SS 8, intermediary device 160, and/or display device 150, for example, for facilitating authentication therebetween. Such communications include messaging (e.g., advertisement, command, or other messaging), message delivery, and analyte data. For example, in certain embodiments, server system 134 may process and exchange messages between SS 8 and display device 150 related to frequency bands, timing of transmissions, security, alarms, and so on. In certain embodiments, server system 134 may also update information stored on SS 8, intermediary device 160, and/or display device 150. In certain embodiments, server system 134 may send/receive information to/from SS 8, intermediary device 160, and/or display device 150 in real-time or sporadically. Further, in certain embodiments, server system 134 may implement cloud computing capabilities for SS 8, intermediary device 160, and/or display device 150.

**FIG. 1B** also illustrates the components of SS 8 in further detail. As shown, in certain embodiments, SS 8 includes analyte sensor 10 coupled to sensor electronics module 12. Sensor electronics module 12 includes sensor measurement circuitry (SMC) 13 that is coupled to analyte sensor 10 for processing and managing sensor data. SMC 13 may also be coupled to processor 11. In some embodiments, processor 11 may perform part or all of the functions of the SMC 13 for obtaining and processing sensor measurement values from analyte sensor 10. Processor 11 may also be coupled to storage 14 and real time clock (RTC) 17 for storing and tracking sensor data. In addition, processor 11 may be further coupled to a connectivity interface 15, which includes a radio unit or transceiver (TRX) 16 for sending sensor data and receiving requests and commands from an external device, such as display device 150 and intermediary device 160. As used herein, the term transceiver generally refers to a device or a collection of devices that enable SS 8 to (e.g., wirelessly) transmit and receive data. SS 8 may further include storage 14 and real time clock (RTC) 17 for storing and tracking sensor data. It is contemplated that, in some embodiments, the SMC 13 may carry out all the functions of the processor 11 and vice versa.

Transceiver 16 may be configured with the necessary hardware and wireless communications protocols for enabling wireless communications between SS 8 and other devices, such as display device 150, intermediary device 160, and/or server system 134. For example, as described above, transceiver 16 may be configured with the necessary hardware and communication protocols to establish a Bluetooth or BLE connection with display device 150 and/or intermediary device 160. As one of ordinary skill in the art appreciates, in such an example, the necessary hardware may include a Bluetooth or BLE security manager and/or other Bluetooth or BLE related hardware/software modules configured for Bluetooth or BLE communications standards. In some embodiments where SS 8 is configured to establish an independent communication path with server system 134, transceiver 16 may be configured with the necessary hardware and communication protocols (e.g., long range wireless cellular communication protocol, such as, Global System for Mobile Communications (GSM), Code Division Multiple Access (CDMA), Long-Term Evolution (LTE), Voice over LTE (VoLTE), 3G, 4G, and 5G communication protocols, WiFi communication protocols, such as 802.11 communication protocols, etc.) for establishing a wireless connection to network 190 to connect with server system 134. As discussed elsewhere, other short range protocols, may also be used for communication between SS 8 and display device 150 and/or intermediary device 160 such as NFC, RFID, etc.

**FIG. 1B** similarly illustrates the components of intermediary device 160 in further detail. As shown, intermediary device 160 includes connectivity interface 147, processor 143, memory 144, and a storage 145. In certain embodiments, the intermediary device 160 additionally includes a display 192 configured to display sensor information and/or analyte data to a user and/or receive inputs from the user. A bus (not shown here) may be used to interconnect the various elements of intermediary device 160 and transfer data between these elements. Connectivity interface 147 includes a transceiver (TRX) 146 used for receiving sensor data from SS 8 and for sending requests, instructions, and/or data to SS 8 as well as display device 150 and/or server system 134. Transceiver 146 is coupled to other elements of intermediary device 160 via connectivity interface 147 and/or the bus. Transceiver 146 may include multiple transceiver modules operable on different wireless standards. For example, transceiver 146 may be configured with one or more communication protocols, such as wireless communication protocol(s) for establishing a wireless communication path with network 190 and/or low range wireless communication protocol(s) (e.g., Bluetooth or BLE) for establishing wireless communication paths 185, 186, and/or 187. Additionally, connectivity interface 147 may in some cases include additional components for controlling radio and/or wired connections, such as baseband and/or Ethernet modems, audio/video codecs, and so on.

**FIG. 1B** similarly illustrates the components of display device 150 in further detail. As shown, display device 150 includes connectivity interface 128, processor 126, memory 127, one or more sensors 163, a display 125 for presenting a graphical user interface (GUI), and a storage 123. A bus (not shown here) may be used to interconnect the various elements of display device 150 and transfer data between these elements. Connectivity interface 128 includes a transceiver (TRX) 129 used for receiving sensor data from SS 8 and for sending requests, instructions, and/or data to SS 8 as well as server system 134. Transceiver 129 is coupled to other elements of display device 150 via connectivity interface 128 and/or the bus. Transceiver 129 may include multiple transceiver modules operable on different wireless standards. For example, transceiver 129 may be configured with one or more communication protocols, such as wireless communication protocol(s) for establishing a wireless communication path with network 190 and/or low range wireless communication protocol(s) (e.g., Bluetooth or BLE) for establishing a wireless communication path 180 with SS 8. Additionally, connectivity interface 128 may in some cases include additional components for controlling radio and/or wired connections, such as baseband and/or Ethernet modems, audio/video codecs, and so on. Sensor(s) 163 may include, but is not limited to, accelerometer(s), gyroscope(s), global positioning system (GPS) sensor(s), heart rate sensor(s), etc. Note that while sensor(s) 163 are shown integral to the display device, in certain embodiments, one or more of sensor(s) 163 may be standalone sensors (e.g., separate from the display device 150).

In some embodiments, when a standardized communication protocol is used between display device 150 and SS 8, commercially available transceiver circuits may be utilized that incorporate processing circuitry to handle low level data communication functions such as the management of data encoding, transmission frequencies, handshake protocols, security, and the like. In such embodiments, processor 126 of display device 150 and/or processor 11 of SS 8 may not need to manage these activities, but instead provide desired data values for transmission, and manage high level functions such as power up or power down, set a rate at which messages are transmitted, and the like. Instructions and data values for performing these high level functions can be provided to the transceiver circuits via a data bus and transfer protocol established by the manufacturer of transceivers 129 and 16. However, in embodiments where a standardized communication protocol is not used between transceivers 129 and 16 (e.g., when non-standardized or modified protocols are used), processors 126 and 11 may be configured to execute instructions associated with proprietary communications protocols (e.g., one or more of the communications protocols described herein) to control and manage their respective transceivers. In addition, when non-standardized or modified protocols are used, customized circuitries may be used to service such protocols.

Processor 126 may include processor sub-modules, including, by way of example, an applications processor that interfaces with and/or controls other elements of display device 150 (e.g., connectivity interface 128, analyte sensor application 121 (hereinafter "sensor application 121"), display 125, sensor(s) 163, memory 127, storage 123, etc.). In certain embodiments, processor 126 is configured to perform functions related to device management, such as, for example, managing lists of available or previously paired devices, information related to network conditions (e.g., link quality and the like), information related to the timing, type, and/or structure of messaging exchanged between SS 8 and display device 150, and so on. Processor 126 may further be configured to receive and process user input, such as, for example, a user's biometric information, such as the user's finger print (e.g., to authorize the user's access to data or to be used for authorization/encryption of data, including analyte data), as well as analyte data.

Processor 126 may include and/or be coupled to circuitry such as logic circuits, memory, a battery and power circuitry, and other circuitry drivers for periphery components and audio components. Processor 126 and any sub-processors thereof may include logic circuits for receiving, processing, and/or storing data received and/or input to display device 150, and data to be transmitted or delivered by display device 150. As described above, processor 126 may be coupled by a bus to display 125, connectivity interface 128, storage 123, etc. Hence, processor 126 may receive and process electrical signals generated by these respective elements and thus perform various functions. By way of example, processor 126 may access stored content from storage 123 and memory 127 at the direction of analyte sensor application 121, and process the stored content to be displayed by display 125. Additionally, processor 126 may process the stored content for transmission via connectivity interface 128 to SS 8, intermediary device 160, and/or server system 134. Display device 150 may include other peripheral components not shown in detail in **FIG. 1B****.**

In certain embodiments, memory 127 may include volatile memory, such as random access memory (RAM) for storing data and/or instructions for software programs and applications, such as analyte sensor application 121. Display 125 presents a GUI associated with operating system 162 and/or analyte sensor application 121. In various embodiments, a user may interact with analyte sensor application 121 via a corresponding GUI presented on display 125. By way of example, display 125 may be a touchscreen display that accepts touch input. Analyte sensor application 121 may process and/or present analyte-related data received by display device 150 and present such data via display 125. Additionally, analyte sensor application 121 may be used to obtain, access, display, control, and/or interface with analyte data and related messaging and processes associated with SS 8 (e.g., and/or any other medical device (e.g., insulin pump or pen) that are communicatively coupled with display device 150), as is described in further detail herein.

Storage 123 may be a non-volatile storage for storing software programs, instructions, data, etc. For example, storage 123 may store analyte sensor application 121 that, when executed using processor 126, for example, receives input (e.g., by a conventional hard/soft key or a touch screen, voice detection, or other input mechanism), and allows a user to interact with the analyte data and related content via display 125. In various embodiments, storage 123 may also store user input data and/or other data collected by display device 150 (e.g., input from other users gathered via analyte sensor application 121). Storage 123 may further be used to store volumes of analyte data received from SS 8 (or any other medical data received from other medical devices (e.g., insulin pump, pen, etc.) for later retrieval and use, e.g., for determining trends and triggering alerts.

As described above, SS 8, in certain embodiments, gathers analyte data from analyte sensor 10 and transmits the same or a modified version of the collected data to display device 150. Data points regarding analyte values may be gathered and transmitted over the life of analyte sensor 10 (e.g., in the range of 1 to 30 days or more). New measurements may be transmitted often enough to adequately monitor glucose levels. In certain embodiments, rather than having the transmission and receiving circuitry of each of SS 8 and display device 150 continuously communicate, SS 8 and display device 150 may regularly and/or periodically establish a communication channel among each other. Thus, in such embodiments, SS 8 may, for example, communicate with display device 150 at predetermined time intervals. The duration of the predetermined time interval can be selected to be long enough so that SS 8 does not consume too much power by transmitting data more frequently than needed, yet frequent enough to provide substantially real-time sensor information (e.g., measured glucose values or analyte data) to display device 150 for output (e.g., via display 125) to the user. While the predetermined time interval is every five minutes in some embodiments, it is appreciated that this time interval can be varied to be any desired length of time. In other embodiments, transceivers 129 and 16 may be continuously communicating. For example, in certain embodiments, transceivers 129 and 16 may establish a session or connection there between and continue to communicate together until the connection is lost.

Analyte sensor application 121 may be downloaded, installed, and initially configured/setup on display device 150. For example, display device 150 may obtain analyte sensor application 121 from server system 134, or from another source, such as an application store or the like, via a network, e.g., network 190. Following installation and setup, analyte sensor application 121 may be configured to access, process, and/or interface with analyte data (e.g., whether stored on server system 134, locally from storage 123, from SS 8, or any other medical device). By way of example, analyte sensor application 121 may present a menu that includes various controls or commands that may be executed in connection with the operation of SS 8, display device 150, one or more other display devices (e.g., display device 110, 130, 140, etc.), and/or one or more other partner devices, such as an insulin pump. For example, analyte sensor application 121 may be used to interface with or control other display and/or partner devices, for example, to deliver or make available thereto analyte data, including for example by receiving/sending analyte data directly to the other display and/or partner device and/or by sending an instruction for SS 8 and the other display and/or partner device to be connected.

After downloading analyte sensor application 121, as one of the initial steps, the user may be directed by analyte sensor application 121 to wirelessly connect display device 150 to the user's SS 8, which the user may have already placed on their body. A wireless communication path 180 between display device 150 and SS 8 allows SS 8 to transmit analyte measurements to display device 150 and for the two devices to engage in any of the other interactions described above.

### EXAMPLE COMMUNICATION PROTOCOL ARCHITECTURE

**FIG. 2** illustrates an example communication protocol architecture 200, according to certain embodiments. In the depicted embodiment, the communication protocol architecture 200 (also referred to as a communication protocol stack) includes an application layer 204, a host layer 208, and a controller layer 210. The application layer 204 is the highest layer(s) of the communication protocol architecture 200 and generally provides application layer services, device roles and modes, and/or connection management. The host layer 208 and controller layer 210 are the lower layer(s) of the communication protocol architecture 200. As used herein, a "lower protocol layer" may refer to the host layer 208 and/or controller layer 210.

The controller layer 210 may provide physical layer (PHY) services, including, for example, analog communication circuitry responsible for translation of digital data over the air. The host layer 208 is generally responsible for tasks, such as (i) inviting, scanning, and creating/maintaining connections, (ii) encapsulating the data received from upper layers and generating packets for transmission via the controller layer 210, (iii) performing packet error-detecting, (iv) encryption/decryption of the communication, (v) segmentation and reassembly operations for packets exchanged between the application layer 204 and the controller layer 210, and the like.

Although not shown in **FIG. 2****,** in certain embodiments, the communication protocol architecture 200 may also include a host controller interface (HCI), which is a thin layer that transports commands and events between the host layer 208 and controller layer 210.

As described in greater detail below, certain aspects described herein provide techniques for implementing data security at the higher layer(s) of the communication stack (e.g., application layer 204) as opposed to at the lower layer(s) of the communication stack (e.g., host layer 208 and/or controller layer 210), in order to address a number of technical issues, including, for example, resource inefficiencies associated with implementing data security at the higher layer(s) of the communication stack.

### EXAMPLE SECURE BROADCAST MESSAGING IN SUPPORT OF GLUCOSE MONITORING

As discussed, in conventional communication protocols used for exchanging analyte data, data security (e.g., integrity, authentication, and encryption) is typically implemented at the lower protocol layer (e.g., host layer 208 and/or controller layer 210) of the communication stack (e.g., communication protocol architecture 200). As noted, however, implementing such data security at the lower protocol layer of the communication stack may introduce a number of technical issues, including, for example, resource inefficiencies and signal loss issues.

Accordingly, to address the challenges associated with providing data security at the lower protocol layer of a communication protocol stack, certain embodiments described herein provide techniques for performing encryption, data integrity, and/or authentication at the application layer (e.g., application layer 204) of a communication protocol stack (e.g., communication protocol architecture 200). For example, the analyte sensor system (e.g., SS 8) and display device (e.g., display device 150) can be configured to perform application-layer security, as opposed to implementing data security at the lower protocol layer.

Additionally, in certain embodiments, the analyte sensor system and display device are configured to communicate only over the three primary invitation channels (as opposed to the thirty-seven data channels). Because, in some instances, even if data security is provided at the application layer instead of the lower protocol layer, if data is exchanged through any of the thirty-seven data channels, then the lower protocol layer is configured to automatically perform pairing, bonding, connecting, and disconnecting as well as provide an additional layer of protocol-based data security. Thus, in order to circumvent the need to pair, bond, connect, and disconnect through the lower protocol layer, the display device and the analyte sensor system can be configured to only communicate over the three primary invitation channels. As such, by performing application layer security and communicating over the primary invitation channels, the analyte sensor system and the display device can securely exchange analyte information without the need to pair, bond, connect, and disconnect, as well as the need to use any security protocols offered by the lower protocol layer.

For example, the analyte sensor system and the display device can establish a secured link by establishing and sharing a shared secret (hereinafter, "secret key") (e.g., a cryptographic key) over the primary invitation channels. The analyte sensor system and the display device can participate in a cryptographic key exchange protocol, such as password authenticated key exchange (PAKE), over the primary invitation channels to establish the secret key. Once the secret key is established and known by both the analyte sensor system and the display device, the analyte sensor system and the display device can securely exchange (e.g., via encryption) the analyte information and/or other information over the primary invitation channels, without the need to pair, bond, connect, and disconnect.

In an exemplary scenario, the patient's analyte sensor system may encrypt analyte information using the secret key and broadcast the encrypted analyte information over the primary invitation channels. The display device may then receive the encrypted analyte information broadcast from the patient's analyte sensor system, and decrypt the encrypted analyte information using the secret key.

Advantageously, by circumventing the need to connect, pair, bond, and disconnect, the patient's analyte sensor system and display device are able to save compute, storage, and battery resources, as well as use less communication resources associated with the data channels. Further, because connecting, pairing, and bonding are not performed, analyte data can be shared by the patient's analyte sensor system with the display device more quickly, resulting in a better user experience. For example, once the patient's analyte sensor system and a display device establish and share a secret key, the patient's analyte sensor system can encrypt analyte data at the application layer and broadcast it through the primary invitation channels, without the need to connect or disconnect with the display device. The display device then receives the encrypted analyte data and decrypts it using the secret key or some version thereof. Note that because the analyte data is encrypted, even if other devices in proximity to the patient's analyte sensor system receive the broadcast analyte data, they would not be able to use or decrypt it without the secret key.

In addition, because data is cached at the application layer, encrypted messages can be resent without the need for re-encrypting. For example, when a message has to be resent by the analyte sensor system, because the cached encrypted message is available, it can be resent to the display device over the primary invitation channels, thereby making communication less compute and power intensive, which in turn, reduces battery consumption of the transmitter of the analyte sensor system.

Further, although certain embodiments herein involve using a reduced number of channels (e.g., three primary invitation channels as opposed to thirty-seven data channels) to communicate data, an increase in signal loss is not expected because, currently, in order to connect, exchange data, and disconnect, a display device must, at least in certain cases, receive an analyte sensor system's invitation data. As such, signal loss associated with the current protocols is already dependent on the three primary invitation channels not being jammed or unable to allow a connection. As such, because the same three primary invitation channels are used to communicate, channel performance can be expected to be as good or better than the current protocols. In particular, if instead of performing a connection and disconnection, a message is resent, a decrease in signal loss will even be expected.

As noted above, although certain embodiments described herein refer to an analyte sensor system (e.g., SS 8) performing one or more techniques described herein for application-layer security and communication over primary invitation channels, it is the transmitter (also referred to as the sensor electronics module) in the analyte sensor system that performs the techniques described herein for application-layer security and communication over primary invitation channels. For example, the transmitter in the analyte sensor system is configured to establish a secret key with a display device (and/or another computing system) over the primary invitation channels, to encrypt data using the secret key at the application layer, and to broadcast the encrypted data over the primary invitation channels.

**FIG. 3** is a sequence diagram 300 illustrating example operations performed by an SS 8 and a display device 150, according to certain embodiments described herein. At step 302, SS 8 and display device 150 engage in a cryptographic key exchange over one or more primary invitation channels. The cryptographic key exchange may involve executing a cryptographic key exchange algorithm, examples of which include, but are not limited to, PAKE protocol, Diffie-Hellman (DH) key exchange algorithm, Elliptic Curve Diffie-Hellman (ECDH), etc.

In particular, in certain embodiments, the cryptographic key exchange algorithm includes a key-agreement protocol. A key agreement protocol is a protocol whereby two or more parties can agree on a shared secret in such a way that both influence the outcome. One example of a key agreement protocol may be an exponential key exchange algorithm, such as the DH key exchange algorithm. DH key exchange is a method of securely exchanging cryptographic keys to establish a secure channel. Different versions of the DH key exchange are also within the scope of the disclosure. For example, in certain embodiments, the cryptographic key exchange algorithm includes ECDH, which is a key agreement protocol that allows two parties, each having an elliptic-curve public-private key pair, to establish a shared secret over an insecure channel.

Therefore, here, using the cryptographic key exchange algorithm, the SS 8 and display device 150 can establish a secret key (also known as a shared secret or encryption key or cryptographic key). For example, the SS 8 and the display device 150 can each establish the secret key by executing the cryptographic key exchange algorithm at the application layer via primary invitation channels. The secret key can be (i) the secret, referred to as "KE," derived by the cryptographic key exchange algorithm (e.g., PAKE) or (ii) derived from "KE." A reference example secret key "KE2," which is derived from "KE," can be generated using the following: $KE 2 = cryptopgraphic - hash \left(KE, 0xdcc0\right)$ where cryptographic-hash is a cryptographic hash function, such as secure hash algorithm 2 (SHA2), secure hash algorithm 3 (SHA3), advanced encryption standard (AES), or some other acceptable function.

After SS 8 and display device 150 complete the execution of the cryptographic key exchange algorithm, each of SS 8 and display device 150 is in possession of the secret key 310 (steps 304 and 306) that is used to encrypt any subsequent data transmitted therebetween. For example, at step 312, SS 8 encrypts "patient data" at the application layer (e.g., application layer 204) using the secret key 310. At step 314, SS 8 broadcasts the encrypted "patient data" over the primary invitation channels. SS 8 may broadcast the encrypted "patient data" without connecting and disconnecting. As such, instead of using power to connect and disconnect, SS 8 can use this power for resending "patient data" if necessary. At step 316, the display device 150 obtains and decrypts the encrypted "patient data" using the secret key 310.

In certain embodiments, SS 8 broadcasts encrypted "patient data" every N seconds (for some positive integer N) over the one or more primary invitation channels. The value of "patient data" can be in any format. An exemplary "patient data" can include one or more blood glucose values or any other analyte values. For example, such blood glucose values can include the current blood glucose value and one or more previous blood glucose values.

In addition to or as an alternative to SS 8 broadcasting encrypted "patient data," the display device 150 can broadcast encrypted data using the secret key 310. For example, at step 318, display device 150 encrypts data at the application layer (e.g., application layer 204) using the secret key 310. At step 320, display device 150 broadcasts the encrypted data over the primary invitation channels. Display device 150 may broadcast the encrypted data without connecting and disconnecting. At step 322, the SS 8 obtains and decrypts the encrypted data using the secret key 310.

In certain embodiments, the encrypted data broadcast by the display device 150 includes a desired "opcode" and/or "transmit response data." For example, display device 150 can broadcast an "opcode" and/or "transmit response data" (e.g., data transmitted in response to analyte data received from SS 8) every K seconds, for some positive integer K. The "opcode" is a command that the display device 150 can send to SS 8. The "opcode" enables the display device 150 to communicate with SS 8 using a pre-programmed protocol where opcode values are mapped to specific higher level functions at SS 8. For example, some opcode values may trigger SS 8 to send data. The value of "transmit response data" can be any data value that the display device 150 is configured to transmit. In an exemplary scenario, the display device 150 can send an opcode value that triggers the patient's SS 8 to broadcast encrypted analyte data. Additionally, upon receiving the encrypted analyte data, the display device 150 can send transmit response data indicating that the encrypted analyte data was received. Thus, the display device 150 can also use techniques herein for application-layer security and communication over primary invitation channels to send information to the SS 8 without having to repeatedly connect and disconnect.

Certain embodiments described herein for performing application-layer security and communicating over primary invitation channels may allow for increased connectivity between one or more devices in an indoor environment, such as a patient's sleeping area (e.g., patient's bedroom), as an illustrative, non-limiting example.

For example, a patient may change their sleeping behavior in a manner that results in decreased connectivity between the patient's display device 150 (e.g., smartphone) and the patient's SS 8. For example, the patient may sleep in a particular position/orientation, such that the transmitter (e.g., transceiver 16) of the patient's SS 8 no longer has a line-of-sight (LOS) to the patient's display device 150. For instance, the transmitter of the SS 8 on the patient's abdomen may not have LOS to the patient's display device 150 on a nightstand when the patient is sleeping on their side and facing away from the nightstand.

In such scenarios, the patient's display device 150 may not receive encrypted data from the SS 8. At the same time, the patient's SS 8 may have LOS to another display device 150 in another location within the environment. In certain embodiments, the other display device 150 may be configured with an analyte sensor application 121 or a similar application to be able to receive and manage the patient's analyte measurements from the SS 8 in a variety of ways.

For example, the patient's SS 8 may have LOS to another user's display device 150 on another nightstand. In such an example, if the other user's display device 150 is in proximity to the patient's SS 8, the other user's display device 150 can receive those messages broadcast from the patient's SS 8. The other user's display device 150 could then send the data to a cloud server (e.g., server system 134), and the patient's display device 150 could download the data from the cloud server. The cloud server can be located in a local (private) cloud environment or public cloud environment. Using a local cloud allows the other user's display device 150 to receive the glucose measurements and transfer them to the local cloud, thereby enabling the patient's display device 150 to download the glucose measurements from the local cloud, even if the patient's display device 150 loses internet connectivity or is otherwise unavailable. In this example, it is beneficial to have the patient's SS 8 broadcast data that is encrypted with a secret key over the primary invitation channel(s), because the patient's SS 8 is not tied to any one display device 150. Instead, any display device 150 that has the secret key and can detect the patient's SS 8 (e.g., the display device 150 is in proximity to the patient's SS 8) will be able to receive the glucose measurements.

**FIG. 4** is a sequence diagram 400 illustrating example operations performed by an SS 8, one or more display devices 150, and a server system 134, according to certain embodiments described herein. Note that one or more of the operations in sequence diagram 400 may be performed after one or more operations in sequence diagram 300. For example, the SS 8 and display device 150-1 (e.g., patient's display device) may have engaged in a cryptographic key exchange over primary invitation channels to establish a secret key 310 (step 302 of sequence diagram 300 in **FIG. 3**).

At step 402, the SS 8 broadcasts encrypted data (e.g., data encrypted using the secret key 310). As noted, in some instances, the transmitter of SS 8 may lose LOS with the patient's display device 150-1. For example, the patient may have changed positions and/or orientation, such that the transmitter no longer has LOS to the patient's display device 150-1. In such instances, the patient's display device 150-1 may not be able to receive encrypted data broadcast from SS 8, e.g., as shown in step 404.

At the same time, the transmitter may have LOS to another user's display device 150-2, such that the display device 150-2 may be able to receive the encrypted data broadcast from SS 8. For example, at step 406, the other user's display device 150-2 receives the encrypted data broadcast from SS 8 over the primary invitation channels. At step 410, the display device 150-2 transmits the encrypted data to server system 134. The display device 150-2 may transmit the encrypted data to the server system 134 using a pre-established secure channel between the display device 150-2 and server system 134. For example, the pre-established secure channel may be an encrypted cellular connection (e.g., 5G, 4G, etc.), encrypted WiFi connection, etc. The server system 134 may be located in a local cloud environment or public cloud environment. At step 412, the server system 134 transmits the encrypted data to the patient's display device 150-1, e.g., over the pre-established secure channel between the server system 134 and patient's display device 150-1. At step 414, the patient's display device 150-1 decrypts the encrypted data using the secret key 310.

Advantageously, broadcasting the patient's encrypted analyte data is beneficial because it allows the other user's display device 150-2 to receive the encrypted analyte data and transfer it to the server system 134, thereby enabling the patient's encrypted analyte data to reach the server system 134, even if the patient's display device 150-1 loses internet connectivity.

Note that, in certain embodiments, the other user's display device 150-2 may be configured to not parse the patient's encrypted analyte data. Instead, the other user' s display device 150-2 may just forward the patient's encrypted analyte data to the server system 134 (without parsing the encrypted analyte data). As shown in sequence diagram 400, for example, after the other user's display device 150-2 receives the encrypted data broadcast from SS 8 (step 406), the display device 150-2 forwards the encrypted data to server system 134 (step 410).

In certain embodiments, however, the other user's display device 150-2 may decrypt the patient's encrypted data using the secret key 310 (step 408), and then re-encrypt (step 416) and upload that data to the server system 134 (step 410). Here, the other user's display device 150-2 may use the secret key 310 previously received from the patient's display device 150-1, SS 8, intermediary device 160, and/or server system 134 to decrypt and re-encrypt the data received from SS 8. For example, the other user's display device 150-2 may use a software application that allows the other user's display device 150-2 to view the patient's encrypted data. For instance, the other user may be a guardian of the patient, family member of the patient, friend of the patient, etc. In addition to viewing the patient's encrypted data, the software application may re-encrypt the data (step 416) and upload the re-encrypted data to the server system 134 (step 410), allowing the patient's display device 150-1 and potentially one or more additional users' display devices to download and view the data (assuming the additional users' display devices have the secret key). Note that techniques for sharing a secret key (e.g., secret key 310) among devices, such as display devices and/or intermediary devices, are disclosed herein.

As noted above, utilizing the lower protocol layer for security involves the use of targeted device lists (e.g., whitelists) that have a limit on the number of devices that can be placed on the list. For example, typically only one device can be placed on the targeted device list. In such cases, when SS 8 pairs and bonds with display device 150-1, the one-device capacity of SS 8's targeted device list is reached. Therefore, in such an example, it may not be possible to use other devices as additional retrieval points. However, the embodiments described herein circumvent the need for a targeted device list and, therefore, allow the other user's display device 150-2 to listen locally for the patient's encrypted data broadcast by SS 8, thereby increasing the likelihood of the patient's encrypted data being eventually received by display device 150-1. In particular, in certain embodiments, the other user's display device 150-2 may use a software application that allows the other user's display device 150-2 to act as an additional retrieval point for the patient's encrypted data.

As discussed, connectivity and signal loss are two challenging issues when exchanging analyte information between analyte sensor systems (e.g., SS 8) and display devices (e.g., display devices 150). These issues are at least partly addressed by changing the communication protocol between the analyte sensor system and display devices as described above, and by optionally adding one or more intermediary devices (e.g., intermediary devices 160) into a patient's environment. For example, more listening devices can be added in the patient's environment to increase the chance or probability of the transmitted analyte data being received by a secondary display device or an intermediary device, should the primary display device not receive the SS 8's transmitted analyte data. An intermediary device may include any computing device that can communicate using a wireless communication protocol, such as Bluetooth Low Energy (BLE), WiFi, cellular communications, etc. Examples of intermediary devices can include, but are not limited to, desktops, wireless print servers, game servers, robot controllers, wireless cameras, etc.

The one or more intermediary devices (not including the patient's display device) can "hear" (e.g., detect) a patient's SS 8, meaning that the one or more intermediary devices can receive encrypted analyte data broadcast from the SS 8 and transmit it to a server (e.g., server system 134, which may be located in on-premise/local cloud or public cloud) and/or to another device that the patient's device is able to communicate with. For example, a battery-operated device or a plugged-in device could be located underneath a patient's bed and can listen to messages from a transmitter located on a patient's stomach. These intermediary device(s) can increase the likelihood that the patient's SS 8 transmitted data is received by a display device and/or intermediary device for a patient that sleeps on their stomach.

In certain embodiments, should a patient want to move about her home or in a hospital without the patient's display device 150, intermediary device(s) located within the home or hospital could listen to the patient's SS 8 and receive messages broadcast from the SS 8. If an intermediary device needs to be able to parse and decrypt received data, then the intermediary device can be configured to obtain the secret key that the SS 8 uses to encrypt (or cryptographically sign) messages.

In such embodiments, the technical problem of connectivity is then reduced to how the secret key can be shared among different intermediary devices. Once the secret key is shared among intermediary devices, any intermediary device that receives data from an analyte sensor system can use the secret key to decrypt that data, as well as encrypt or sign data. The secret key shared among intermediary devices creates the possibility for creating mesh networks. For example, assume there are intermediary devices in a home or hospital environment that are all capable of using the described protocol. If these intermediary devices create acceptable coverage within the home or hospital environment, then a patient may move freely through her home or hospital environment where these intermediary devices capture all of her blood glucose data. The patient's analyte sensor system would broadcast the patient's data, allowing any intermediary device that is able to detect the patient's analyte sensor system to receive the broadcast data. For storage, these intermediary devices could upload the data to a cloud platform, and they could also store the data locally. If a patient needed decision support or automated hybrid-closed loop control, this support could come from the cloud platform or via a local cloud. Decision support, for example, may include health outcomes, diet recommendations, exercise recommendations, etc. Automated hybrid-closed loop control may include, for example, automated control of insulin delivery via an insulin pump based on real-time glucose data.

Accordingly, in the aforementioned embodiments, the benefit of using an application layer generated secret key is that it allows a user (e.g., patient) to share the secret key with other devices (e.g., intermediary devices and/or display devices) and also to control who the secret key is shared with (as opposed to BLE layer encryption keys that are generated during communication with a single device). By being able to generate a secret key and share it, the patient's analyte sensor system can broadcast encrypted analyte data without "worrying" about which devices receive the encrypted analyte data. The patient's analyte sensor system can also broadcast encrypted analyte data without having to establish a BLE connection with specific devices. For example, the patient's analyte sensor system can just broadcast encrypted analyte data over the primary invitation channels and trust that, when the right devices detect the broadcast, the devices will be able to decrypt and use the analyte data.

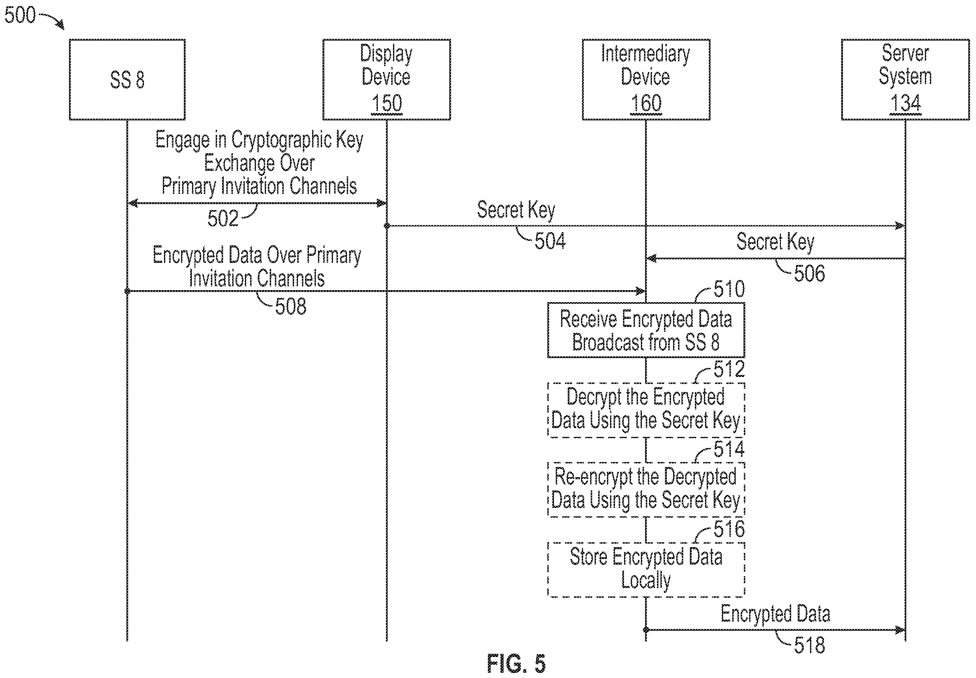

**FIG. 5** is a sequence diagram 500 illustrating example operations performed by an SS 8, intermediary device 160, a display device 150, and a server system 134, according to certain embodiments disclosed herein. In certain embodiments, the operations in sequence diagram 500 may be performed by SS 8, intermediary device 160, display device 150, and server system 134 in order to share a secret key with one or more intermediary devices 160.

At step 502, the SS 8 and display device 150 (e.g., patient's display device) engage in a cryptographic key exchange over primary invitation channels to establish a secret key (e.g. secret key 310). For example, after executing the cryptographic key exchange, both SS 8 and the patient's display device 150 may possess the secret key.

At step 504, the patient's display device 150 transmits the secret key to the server system 134, for example, using a pre-established secure channel between the patient's display device 150 and server system 134. That is, the patient's display device 150 and sever system 134 may already have a secured (e.g., encrypted) channel established, such that the secret key is securely transmitted to the server system 134 from the patient's display device 150.

At step 506, the server system 134 transmits the secret key to intermediary device 160. Here, the server system 134 may also transmit the secret key to the intermediary device 160 over a pre-established secure channel between the server system 134 and intermediary device 160. That is, the intermediary device 160 and sever system 134 may already have a secured channel established, such that the secret key is securely transmitted by the server system 134 to the intermediary device 160.

At step 508, the SS 8 broadcasts encrypted data (e.g., data encrypted using the secret key) over one or more primary invitation channels. The patient's display device 150 may or may not detect the broadcast (e.g., the patient's display device 150 may or may not be in proximity to the patient's transmitter). However, at step 510, the intermediary device 160 is able to detect the broadcast and receives the encrypted data broadcast from SS 8.

In certain embodiments, the intermediary device 160 may not need the secret key that is shared between SS 8 and the patient's display device 150. For example, the intermediary device 160 may be used to pass encrypted data to the server system 134 and may not necessarily need to decrypt the patient's encrypted data. As shown in step 518, for example, the intermediary device 160 may transmit the encrypted data to server system 134, e.g., after receiving the encrypted data in step 510.

On the other hand, in certain embodiments, if an intermediary device 160 needs to decrypt the patient's encrypted data (e.g., for display to a user, such as a patient), then the intermediary device 160 can decrypt the encrypted data using the secret key (step 512), display the data, and re-encrypt the decrypted data using the secret key (step 514). In certain embodiments, the intermediary device 160 can store the encrypted data locally (e.g., in storage 145) (step 516). Storing the encrypted data locally may be beneficial because if a patient needed decision support (e.g., health outcomes, diet recommendations, exercise recommendations, etc.) or automated hybrid-closed loop control (e.g., automated control of insulin delivery via an insulin pump based on real-time glucose data), such support could come from the cloud platform or via the local cloud and use the patient's locally stored data.

Alternatively, in certain embodiments, the intermediary device 160 may forward the encrypted data to the server system 134 (step 518), and download data that can be decrypted using the secret key received from the server system 134. The intermediary device 160 can then display the decrypted data.

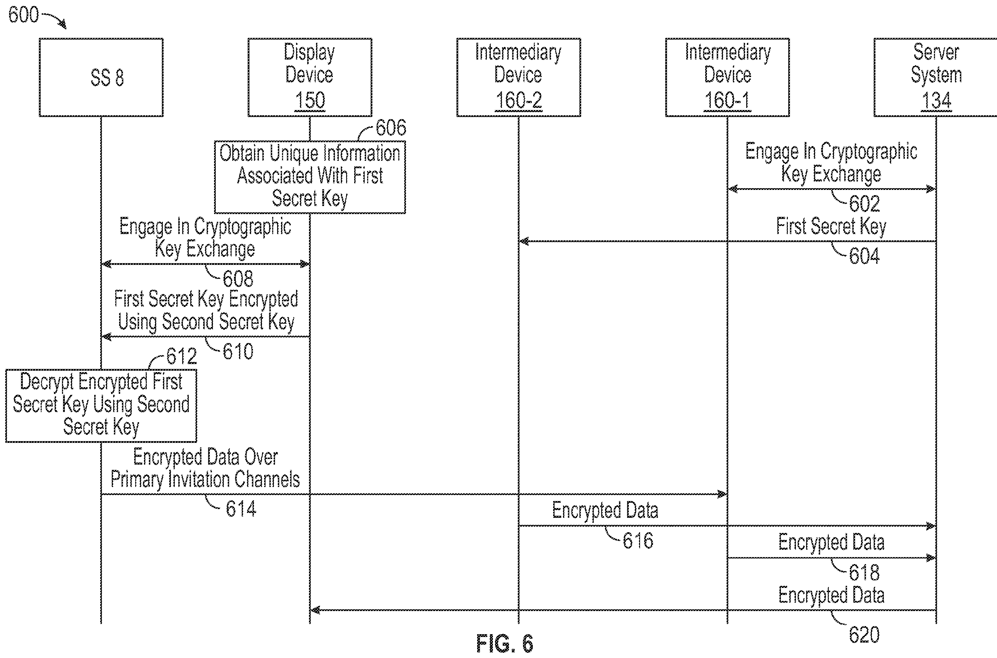

**FIG. 6** is a sequence diagram 600 illustrating example operations performed by an SS 8, one or more intermediary devices 160, a display device 150, and a server system 134, according to certain embodiments disclosed herein. In certain embodiments, the operations in sequence diagram 600 may be performed by SS 8, intermediary device(s) 160, display device 150, and server system 134 in order to share a secret key with SS 8 (more specifically, the patient's transmitter).

In many environments that a patient may visit (e.g., hospital, clinic, etc.), such environments may already have one or more intermediary devices (e.g., intermediary devices 160) disposed in various locations within the environment. Additionally, such intermediary devices may already have a secured channel established between the intermediary devices and a server (e.g., server system 134). In cases where a pre-established channel exists between intermediary devices and a server, a secret key can be set up between a first intermediary device and the server using a cryptographic algorithm, such as ECDH, and communicated to the remaining intermediary devices over the secured channel.

For example, at step 602, the intermediary device 160-1 and server system 134 engage in a cryptographic key exchange to establish a first secret key. For example, after executing the cryptographic key exchange, both server system 134 and intermediary device 160-1 may possess the secret key.

At step 604, server system 134 transmits the first secret key to intermediary device 160-2 (e.g., one or more remaining intermediary devices). The first secret key may be transmitted over the pre-established secured channel between intermediary device 160-2 and server system 134.

To get the first secret key on SS 8, the patient's display device 150 can be configured to obtain unique information associated with the first secret key from the environment (e.g., hospital) (step 606). In an exemplary embodiment, a unique quick response (QR) code can be generated for the patient that represents the first secret key (e.g., server-shared secret key). In such an embodiment, no other patient may be able to see the generated QR code. In certain embodiments, at step 606, the patient's display device 50 can take a picture of the QR code and can communicate the value of the QR code to the patient's SS 8, e.g., using a second secret key.

For example, at step 608, the patient's display device 150 and SS 8 may engage in a cryptographic key exchange over primary invitation channels to establish the second secret key. At step 610, the display device 150 broadcasts an encrypted first secret key (e.g., first secret key encrypted using the second secret key). At step 612, the SS 8 obtains and decrypts the encrypted first secret key using the second secret key.

At step 614, the SS 8 broadcasts encrypted data (e.g., data encrypted using the first secret key) over one or more primary invitation channels. As noted, the patient's display device 150 may or may not detect the broadcast from SS 8. However, at step 616, the intermediary device 160-2 detects the broadcast, receives the encrypted data, and transmits the encrypted data to the server system 134, e.g., over the pre-established secure channel between the intermediary device 160-2 and server system 134. Additionally or alternatively, at step 618, the intermediary device 160-1 may detect the broadcast, receive the encrypted data, and transmit the encrypted data to the server system 134, e.g., over the pre-established secure channel between the intermediary device 160-1 and server system 134.

At step 620, the server system 134 transmits the encrypted data to the patient's display device, e.g., over a pre-established secure channel between the display device 150 and server system 134.

In certain embodiments, the environment in which the patient is located, such as a hospital, can implement key rotation by generating new QR codes and enrolling new patients' SS 8 with the new QR codes. Old patients' SS 8 using the previous key (e.g., first secret key) may still work until the previous key is retired.

In certain embodiments, to enable end-to-end encryption between the patient's SS 8 and the server system 134, the secret key (e.g., first secret key) shared between the intermediary devices 160 and server system 134 may not be the same one that is represented in a QR code. For example, in such embodiments, intermediary devices may not have access to the shared secret key on the QR codes.

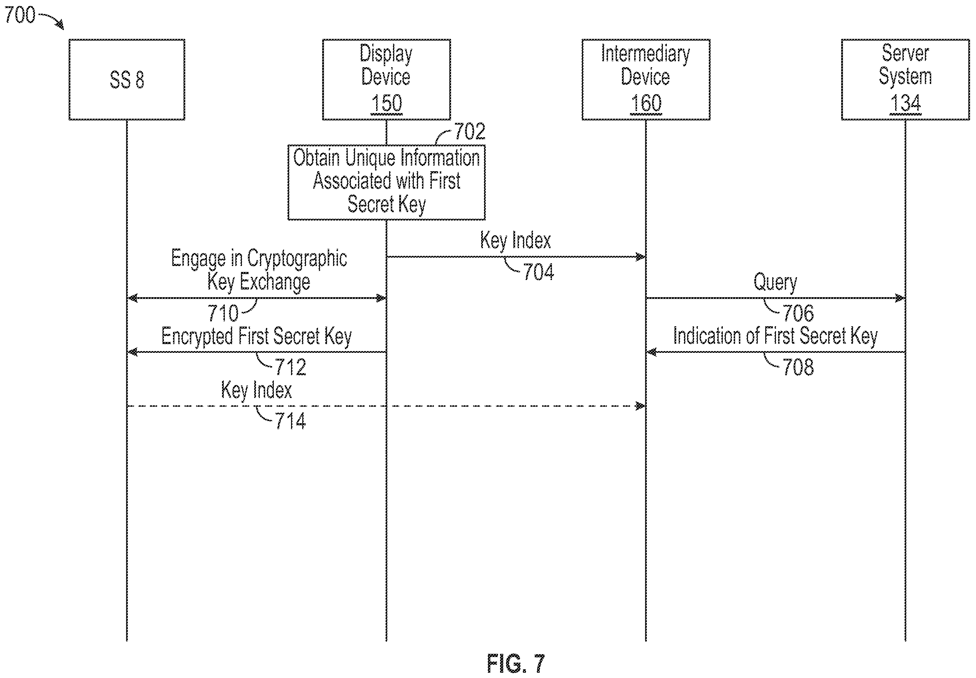

**FIG. 7** is a sequence diagram 700 illustrating example operations performed by an SS 8, an intermediary device 160, a display device 150, and a server system 134, according to certain embodiments disclosed herein. In certain embodiments, the operations in sequence diagram 700 may be performed by SS 8, intermediary device 160, display device 150, and server system 134 in order to share a secret key with an intermediary device 160.

At step 702, the patient's display device 150 obtains unique information (e.g., QR code) associated with a first secret key. In certain embodiments, if the patient wishes to share the first secret key represented in a QR code, the patient's SS 8 or the patient's display device 150 could transmit a key index to the intermediary device 160. At step 704, the patient's display device transmits a key index to the intermediary device 160. At step 706, the intermediary device 160 transmits, to server system 134, a query for the secret key (e.g., first secret key) associated with the key index value received from the patient's SS 8 or the patient's display device 150.

At step 708, server system 134 transmits an indication of the first secret key to the intermediary device 160, e.g., via a pre-established secure channel. In certain embodiments, the intermediary device 160 can cache key index values to avoid asking server system 134 to perform a look-up of the key index value that it received from the patient's transmitter.

In certain embodiments, the patient's SS 8 can also transmit a key index associated with the unique information (e.g., QR code) to the intermediary device 160. As shown, at step 710, the patient's SS 8 and patient's display device 150 engage in a cryptographic key exchange over primary invitation channels to establish a second secret key. At step 712, the patient's display device transmits an encrypted first secret key (e.g., first secret key encrypted with the second secret key) to the patient's SS 8. At step 714, the patient's SS 8 may decrypt the encrypted first secret key using the second secret key and transmit the key index to the intermediary device 160.

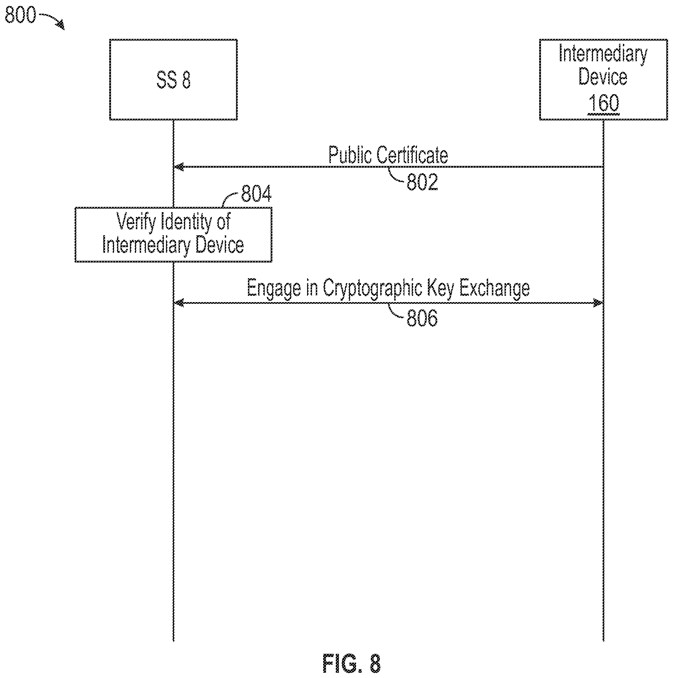

**FIG. 8** is a sequence diagram 800 illustrating example operations performed by an SS 8 and an intermediary device 160, according to certain embodiments disclosed herein. In certain embodiments, the operations in sequence diagram 800 may be performed by SS 8 and intermediary device 160 in order to share a secret key with the patient's SS 8.

At step 802, the intermediary device 160 transmits its public certificate to the patient's SS 8, e.g., over an insecure channel. At step 804, the SS 8 cryptographically verifies the identity of the intermediary device 160. If SS 8 can cryptographically verify the signature on the certificate (e.g., all the way to the root of trust), then SS 8 and the intermediary device 160 can communicate and establish a secret key using a cryptographic key exchange, such as ECDH (step 806).

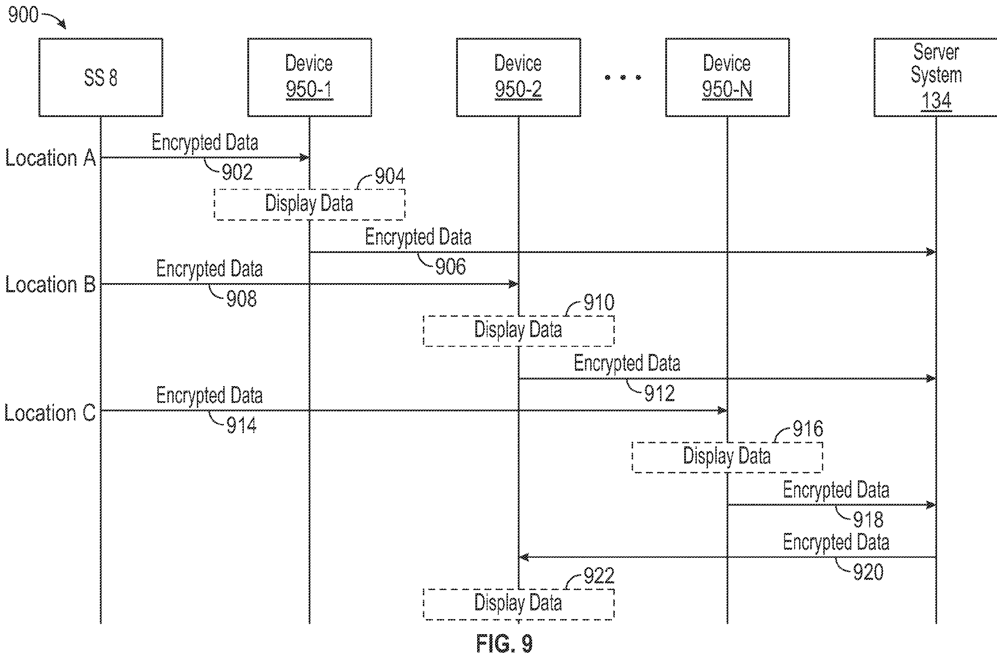

**FIG. 9** is a sequence diagram 900 illustrating example operations performed by an SS 8, one or more devices 950, and a server system 134, according to certain embodiments described herein. Note that one or more of the operations in sequence diagram 900 may assume that the devices 950 are in possession of a secret key used for encrypting the patient's analyte data. The devices 950 1-N may include display devices 150, intermediary devices 160, or combinations thereof.

As discussed, a patient's environment (e.g., patient's home, patient's car, patient's workplace, etc.) may include multiple devices 950 capable of receiving the patient's encrypted data broadcast by SS 8. Such devices 950 can include display devices 150, intermediary devices 160, or combinations thereof.

In certain embodiments, the patient may experience near full connectivity as the patient transitions to different locations. For example, at step 902, SS 8 broadcasts encrypted data when the patient is in location A (e.g., patient's home). The encrypted data broadcast at step 902 may be received by at least one device 950-1 located in location A. The SS 8 may broadcast the encrypted data using short range wireless communications, such as BLE. At step 904, the device 950-1 can decrypt and display the data. Additionally, at step 906, the device 950-1 can transmit the encrypted data to server system 134, e.g., over a pre-established secure channel between device 950-1 and server system 134. For example, the device 950-1 may transmit the encrypted data to the server system 134 using Bluetooth or cellular communications.

When the patient leaves location A (e.g., patient's home) and enters location B (e.g., patient's car), the device 950-2 in location B can continuously monitor for the encrypted data (e.g., blood glucose data) broadcast by SS 8. For example, at step 908, SS 8 broadcasts encrypted data, and the encrypted data may be received by device 950-2 located in location B. At step 910, the device 950-2 can decrypt and display the data. Additionally, at step 912, the device 950-2 can transmit the encrypted data to server system 134, e.g., over a pre-established secure channel between device 950-2 and server system 134.

In an exemplary location B, such as the patient's car (or vehicle), the device 950-2 may possess the secret key in order to decrypt and view the encrypted data broadcast from SS 8. In certain embodiments, the patient's car may connect to the patient's SS 8 directly via local communication connectivity (e.g., via Bluetooth, cellular communications, etc.), via cloud connectivity, or a combination thereof. The car's system (e.g., device 950-2) may display the data and/or upload the data to server system 134 (e.g., via LTE, 5G, or satellite). In another example, the patient's display device could upload data to server system 134, and the patient's car could download that data from server system 134 (step 920). The patient's car can then display the data downloaded from the server system 134 (step 922). The data upload and data download can be performed using a cellular or satellite network.

In certain embodiments, the patient's car may also perform computation on behalf of a patient's display device. For example, the patient's display device could offload computation to a car or to the cloud. The patient's car can then present the patient's data on a display in the car, saving battery power on the patient's display device.

When the patient leaves location B (e.g., patient's car), there may be a brief period of time whether the encrypted data broadcast by SS 8 may not be received by a device 950 capable of detecting SS 8. However, when the patient enters location C (e.g., patient's workplace), the device 950-3 in location C may monitor for the encrypted data (e.g., blood glucose data) broadcast by SS 8. For example, at step 914, SS 8 broadcasts encrypted data, and the encrypted data may be received by device 950-N located in location C. At step 916, the device 950-N can decrypt and display the data. Additionally, at step 918, the device 950-N can transmit the encrypted data to server system 134, e.g., over a pre-established secure channel between device 950-N and server system 134.

When the patient returns to location A (e.g., patient's home), the device 950-1 in location A may listen to the encrypted data (e.g., blood glucose data) broadcast by SS 8. In this manner, the patient may experience near full connectivity between the patient's SS 8 and other devices over time in different locations.

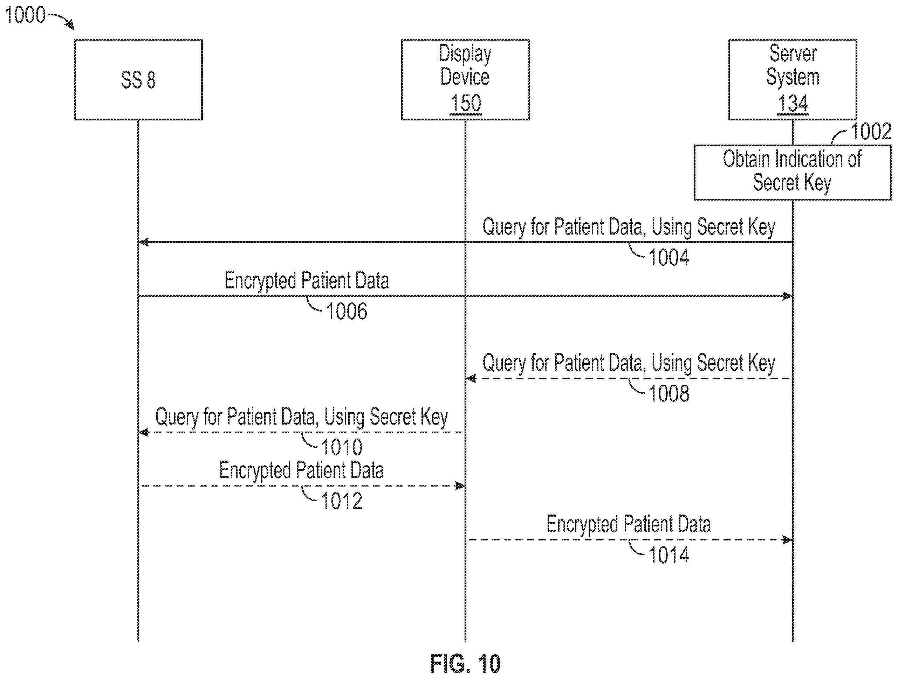

**FIG. 10** is a sequence diagram 1000 illustrating example operations performed by an SS 8, a display device 150, and a server system 134, according to certain embodiments described herein.

At step 1002, the server system 134 obtains an indication of a secret key. In an exemplary embodiment, the server system 134 is used by a healthcare provider to scan a code on a patient's mobile application that has the secret key used by SS 8 to encrypt patient data. After scanning the code, the server system 134 can obtain the secret key and use the secret key to directly query the patient's SS 8 for previous data (e.g., last 24 hours or some other time period) (step 1004). At step 1006, SS 8 transmits the encrypted patient data (e.g., patient data encrypted using the secret key) to the server system.

In certain embodiments, server system 134 can directly query the patient's SS 8 without interacting with the patient's mobile application on the patient's display device 150 (step 1004), for example, using Bluetooth, cellular communications, etc. In certain embodiments, server system 134 can interact with the patient's mobile application on the patient's display device 150 to retrieve the data from the patient's SS 8. For example, at step 1008, server system 134 can transmit the query to the patient's display device 150. At step 1010, display device 150 forwards the query to the patient's SS 8. At step 1012, the patient's SS 8 transmits the encrypted patient data to the patient's display device 150, and at step 1014, the patient's display device 150 transmits the encrypted data to server system 134.

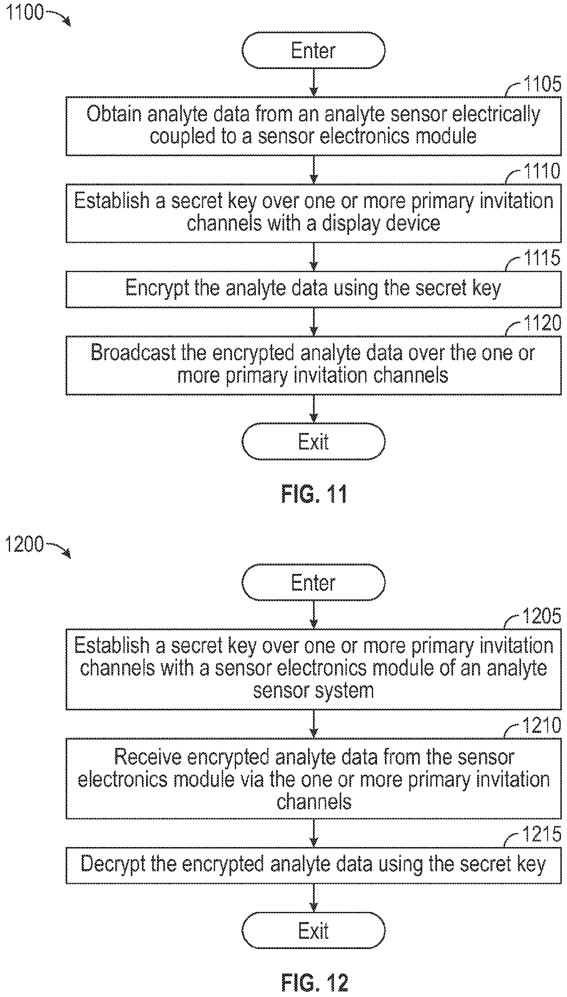

**FIG. 11** is a flow diagram illustrating example operations 1100 for communicating analyte information of a user (e.g., a patient), according to certain embodiments described herein. The operations 1100 may be performed by a sensor electronics module (e.g., sensor electronics module 12) of analyte sensor system (e.g., SS 8).

At operation 1105, the sensor electronics module obtains analyte data from an analyte sensor (e.g., analyte sensor 10) electrically coupled to the sensor electronics module.

At operation 1110, the sensor electronics module establishes a secret key (e.g., secret key 310) over one or more primary invitation channels with a display device (e.g., display device 150). For example, the sensor electronics module and the display device may execute a cryptographic key exchange algorithm at the application layer via the one or more primary invitation channels to establish the secret key.

At operation 1115, the sensor electronics module encrypts the analyte data using the secret key. For example, the sensor electronics module may encrypt the analyte data at the application layer using the secret key.

At operation 1120, the sensor electronics module broadcasts the encrypted analyte data over the one or more primary invitation channels.

**FIG. 12** is a flow diagram illustrating example operations 1200 for receiving analyte information of a user (e.g., a patient), according to certain embodiments described herein. The operations 1200 may be performed by a display device (e.g., display device 150).

At operation 1205, the display device establishes a secret key (e.g., secret key 310) over one or more primary invitation channels with a sensor electronics module (e.g., sensor electronics module 12) of an analyte sensor system (e.g., analyte sensor system 8). For example, the sensor electronics module and the display device may execute a cryptographic key exchange algorithm at the application layer via the one or more primary invitation channels to establish the secret key.

At 1210, the display device receives encrypted analyte data from the sensor electronics module via the one or more primary invitation channels.

At 1215, the display device decrypts the encrypted analyte data using the secret key.

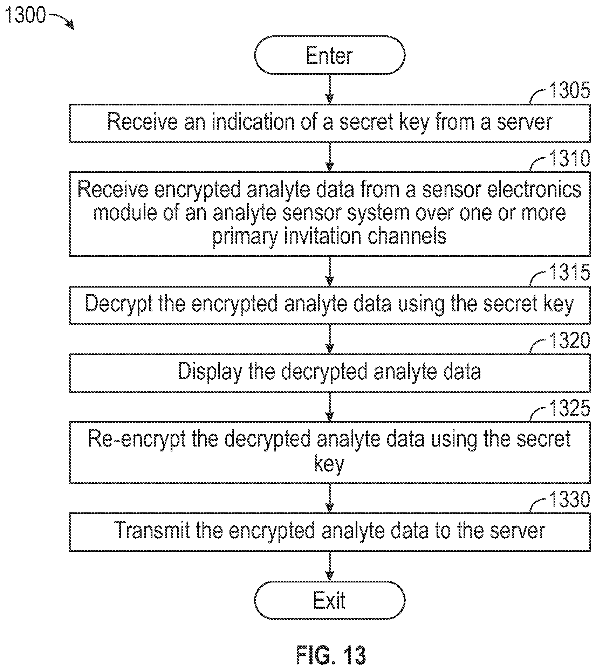

**FIG. 13** is a flow diagram illustrating example operations 1300 for receiving analyte information of a user (e.g., a patient), according to certain embodiments described herein. The operations 1300 may be performed by an intermediary device (e.g., intermediary device 160).

At operation 1305, the intermediary device receives an indication of a secret key from a server (e.g., server system 134). The intermediary device may receive the indication of the secret key via a secure channel established between the intermediary device and the server.

At operation 1310, the intermediary device receives encrypted analyte data from a sensor electronics module (e.g., sensor electronics module 12) of an analyte sensor system (e.g., analyte sensor system 8) over one or more primary invitation channels.

At operation 1315, the intermediary device decrypts the encrypted analyte data using the secret key.

At operation 1320, the intermediary device displays the decrypted analyte data.

At operation 1325, the intermediary device re-encrypts the decrypted analyte data using the secret key.

At operation 1330, the intermediary device transmits the encrypted analyte data to the server, e.g., over the secure channel established between the intermediary device and the server.

Advantageously, performing application-layer security and communicating over the three primary invitation channels using the techniques described herein can reduce consumption of resources (e.g., computer resources, memory resources, etc.) by the transmitter, since the transmitter can avoid repeatedly connecting and disconnecting to send analyte information. Reducing consumption of resources, in turn, reduces battery consumption, which is critical to extending the operational life of the transmitter.

As used herein, "a processor," "at least one processor," or "one or more processors" generally refers to a single processor configured to perform one or multiple operations or multiple processors configured to collectively perform one or more operations. In the case of multiple processors, performance of the one or more operations could be divided amongst different processors, though one processor may perform multiple operations, and multiple processors could collectively perform a single operation. Similarly, "a memory," "at least one memory," or "one or more memories" generally refers to a single memory configured to store data and/or instructions or multiple memories configured to collectively store data and/or instructions.

Each of these non-limiting examples can stand on its own or can be combined in various permutations or combinations with one or more of the other examples. The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Geometric terms, such as "parallel", "perpendicular", "round", or "square", are not intended to require absolute mathematical precision, unless the context indicates otherwise. Instead, such geometric terms allow for variations due to manufacturing or equivalent functions. For example, if an element is described as "round" or "generally round", a component that is not precisely circular (e.g., one that is slightly oblong or is a many-sided polygon) is still encompassed by this description.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

The disclosure in the application also includes the following numbered clauses:
1. A computer-implemented method for communicating analyte data performed by a sensor electronics module of an analyte sensor system, comprising:
   obtaining analyte data from an analyte sensor electrically coupled to the sensor electronics module;
   establishing a secret key with a display device over one or more primary invitation channels;
   encrypting the analyte data using the secret key; and
   broadcasting the encrypted analyte data over the one or more primary invitation channels.
2. The computer-implemented method of clause 1, wherein establishing the secret key comprises participating in a cryptographic key exchange with the display device over the one or more primary invitation channels.
3. The computer-implemented method of clause 2, wherein participating in the cryptographic key exchange comprises executing a cryptographic key exchange algorithm at an application layer of a communication protocol stack at the sensor electronics module.
4. The computer-implemented method of clause 1, wherein the analyte data is encrypted at an application layer of a communication protocol stack at the sensor electronics module.
5. The computer-implemented method of clause 1, further comprising:
   receiving encrypted data from the display device over the one or more primary invitation channels; and
   decrypting the encrypted data using the secret key.
6. The computer-implemented method of clause 5, wherein the decrypted data comprises an opcode.
7. The computer-implemented method of clause 6, wherein a value of the opcode triggers the broadcasting of the encrypted analyte data over the one or more primary invitation channels.
8. The computer-implemented method of clause 1, wherein the encrypted analyte data is broadcast without establishing a connection with the display device.
9. An analyte sensor system comprising:
   an analyte sensor; and
   a sensor electronics module electrically coupled to the analyte sensor and configured to perform an operation comprising:
      obtaining analyte data from the analyte sensor;
      establishing a secret key with a display device over one or more primary invitation channels;
      encrypting the analyte data using the secret key; and
      broadcasting the encrypted analyte data over the one or more primary invitation channels.
10. The analyte sensor system of clause 9, wherein establishing the secret key comprises participating in a cryptographic key exchange with the display device over the one or more primary invitation channels.
11. The analyte sensor system of clause 10, wherein participating in the cryptographic key exchange comprises executing a cryptographic key exchange algorithm at an application layer of a communication protocol stack at the sensor electronics module.
12. The analyte sensor system of clause 9, wherein the analyte data is encrypted at an application layer of a communication protocol stack at the sensor electronics module.
13. The analyte sensor system of clause 9, wherein the encrypted analyte data is broadcast without establishing a connection with the display device.
14. A computer-implemented method for communicating analyte data performed by a display device, comprising:
   establishing a secret key with a sensor electronics module of an analyte sensor system over one or more primary invitation channels;
   receiving encrypted analyte data from the sensor electronics module over the one or more primary invitation channels; and
   decrypting the encrypted analyte data using the secret key.
15. The computer-implemented method of clause 14, further comprising displaying the decrypted analyte data.
16. The computer-implemented method of clause 14, further comprising:
   re-encrypting the decrypted analyte data using the secret key; and
   transmitting the re-encrypted analyte data to a server over a secure channel established between the display device and the server.
17. The computer-implemented method of clause 14, further comprising:
   encrypting data using the secret key; and
   broadcasting the encrypted data over the one or more primary invitation channels.
18. The computer-implemented method of clause 17, wherein the data is encrypted at an application layer of a communication protocol stack at the display device.
19. The computer-implemented method of clause 17, wherein the encrypted data is broadcast in response to receiving the encrypted analyte data from the sensor electronics module.
20. The computer-implemented method of clause 17, wherein the encrypted analyte data is received in response to the encrypted data being broadcast over the one or more primary invitation channels.
21. The computer-implemented method of clause 14, wherein establishing the secret key comprises participating in a cryptographic key exchange with the sensor electronics module over the one or more primary invitation channels.
22. The computer-implemented method of clause 21, wherein participating in the cryptographic key exchange comprises executing a cryptographic key exchange algorithm at an application layer of a communication protocol stack at the display device.
23. The computer-implemented method of clause 14, wherein the encrypted analyte data is received from the sensor electronics module without establishing a connection with the sensor electronics module.
24. A computer-implemented method for communicating analyte data performed by a display device, comprising:
   establishing a secret key with a sensor electronics module of an analyte sensor system over one or more primary invitation channels;
   receiving, at a first point in time, encrypted first analyte data from the sensor electronics module over the one or more primary invitation channels;
   decrypting the encrypted first analyte data using the secret key;
   receiving, at a second point in time subsequent to the first point in time, encrypted second analyte data associated with the analyte sensor system from a server; and
   decrypting the encrypted second analyte data using the secret key.
25. The computer-implemented method of clause 24, wherein the encrypted second analyte data is received over a secure channel established between the display device and the server.
26. The computer-implemented method of clause 24, wherein the display device is unable to receive the encrypted second analyte data from the sensor electronics module at the second point in time.
27. The computer-implemented method of clause 26, wherein the display device is unable to receive the encrypted second analyte data from the sensor electronics module at the second point in time due to losing a line-of-sight (LOS) to the sensor electronics module at the second point in time.
28. A computer-implemented method for communicating analyte data performed by an intermediary device, comprising:
   receiving an indication of a secret key from a server, the secret key being associated with a sensor electronics module of an analyte sensor system;
   receiving encrypted analyte data from the sensor electronics module over one or more primary invitation channels;
   decrypting the encrypted analyte data using the secret key;
   displaying the decrypted analyte data;
   re-encrypting the decrypted analyte data using the secret key; and
   transmitting the re-encrypted analyte data to the server over a secure channel established between the intermediary device and the server.
29. The computer-implemented method of clause 28, wherein the indication of the secret key is received over the secure channel established between the intermediary device and the server.
30. A computer-implemented method for communicating analyte data performed by an intermediary device, comprising:
   receiving encrypted analyte data from a sensor electronics module of an analyte sensor system over one or more primary invitation channels; and
   transmitting the encrypted analyte data to a server over a secure channel established between the intermediary device and the server.
31. The computer-implemented method of clause 30, further comprising storing the encrypted analyte data in a storage location local to the intermediary device.
32. A computer-implemented method for communicating analyte data performed by a server, comprising:
   establishing a secret key with a first intermediary device;
   transmitting an indication of the secret key to at least one second intermediary device;
   receiving encrypted analyte data associated with a sensor electronics module of an analyte sensor system from at least one of the first intermediary device or the second intermediary device, the analyte data being encrypted with the secret key; and
   transmitting the encrypted analyte data to a display device.
33. The computer-implemented method of clause 32, wherein the encrypted analyte data is transmitted to the display device over a secure channel established between the server and the display device.
34. The computer-implemented method of clause 32, wherein establishing the secret key comprises participating in a cryptographic key exchange with the first intermediary device.
35. A computer-implemented method for communicating analyte data performed by a sensor electronics module of an analyte sensor system, comprising:
   obtaining analyte data from an analyte sensor electrically coupled to the sensor electronics module;
   establishing a secret key with a display device, based on executing a cryptographic key exchange algorithm at an application layer of a communication protocol stack at the sensor electronics module;
   encrypting the analyte data using the secret key; and
   transmitting the encrypted analyte data.
36. The computer-implemented method of clause 35, wherein the analyte data is encrypted at the application layer of the communication protocol stack at the sensor electronics module.
37. A system comprising:
   an analyte sensor;
   a first display device;
   a server;
   a sensor electronics module configured to:
      obtain analyte data from the analyte sensor;
      establish a secret key with the first display device over one or more primary invitation channels;
      encrypt the analyte data using the secret key; and
      broadcast the encrypted analyte data over the one or more primary invitation channels; and
   a second display device configured to:
      receive the encrypted analyte data broadcast over the one or more primary invitation channels; and
      transmit the encrypted analyte data to the server over a secure channel established between the second display device and the server, wherein:
         the server is configured to transmit the encrypted analyte data to the first display device over a secure channel established between the first display device and the server; and
         the first display device is configured to:
            receive the encrypted analyte data transmitted from the server;
            decrypt the encrypted analyte data using the secret key; and
            display the decrypted analyte data.
38. The system of clause 37, wherein:
   the encrypted analyte data is received by the second display device at a first point in time; and
   the first display device is unable to receive the encrypted analyte data broadcast over the one or more primary invitation channels at the first point in time.
39. The system of clause 38, wherein the first display device is unable to receive the encrypted analyte data at the first point in time due to losing a line-of-sight (LOS) to the sensor electronics module at the first point in time.
40. The system of clause 37, wherein, in order to establish the secret key with the first display device, the sensor electronics module is configured to participate in a cryptographic key exchange with the first display device over the one or more primary invitation channels.

## Claims

1. A computer-implemented method for communicating analyte data performed by an intermediary device, comprising:
receiving an indication of a secret key from a server, the secret key being associated with a sensor electronics module of an analyte sensor system;
receiving encrypted analyte data from the sensor electronics module over one or more primary invitation channels;
decrypting the encrypted analyte data using the secret key;
re-encrypting the decrypted analyte data using the secret key; and
transmitting the re-encrypted analyte data to the server over a secure channel established between the intermediary device and the server.

2. The computer-implemented method of claim 1, wherein the indication of the secret key is received over the secure channel established between the intermediary device and the server.

3. The computer-implemented method of claim 1, further comprising:
displaying the decrypted analyte data on the intermediary device.

4. The computer-implemented method of claim 1, further comprising:
displaying the decrypted analyte data on a display device.

5. The computer-implemented method of claim 4, wherein the secret key has been established between the sensor electronics module of the analyte sensor system and the display device via a cryptographic key exchange.

6. The computer-implemented method of claim 5, wherein the cryptographic key exchange is executed at an application layer of a communication protocol stack at the sensor electronics module.

7. The computer-implemented method of claim 1, wherein the encrypted analyte data has been encrypted at an application layer of a communication protocol stack at the sensor electronics module.

8. An intermediary device, comprising:
one or more processors configured to:
receive an indication of a secret key from a server, the secret key being associated with a sensor electronics module of an analyte sensor system;
receive encrypted analyte data from the sensor electronics module over one or more primary invitation channels;
decrypt the encrypted analyte data using the secret key;
re-encrypt the decrypted analyte data using the secret key; and
transmit the re-encrypted analyte data to the server over a secure channel established between the intermediary device and the server.

9. The intermediary device of claim 8, wherein the indication of the secret key is received over the secure channel established between the intermediary device and the server.

10. The intermediary device of claim 8, wherein the one or more processors are further configured to display the decrypted analyte data on the intermediary device.

11. The intermediary device of claim 8, wherein the encrypted analyte data is broadcast and received without establishing and maintaining a connection between the intermediary device and the sensor electronics module of the analyte sensor system.

12. The intermediary device of claim 8, wherein the encrypted analyte data is received over one or more advertisement channels.
